# EUROPEAN PATENT APPLICATION

(11) **EP 1 553 414 A1**
(43) Date of publication of application: **13.07.2005**
(21) Application number: 05005066.5
(22) Date of filing: 13.04.1999
(51) Int. Cl.: G01N 33/53, A61K 38/00, A61P 35/00, A61K 39/395

(54) **Methods for the diagnosis and treatment of metastatic prostate tumors**

(62) Divisional of application: 99918516.8
(71) Applicant: Medarex, Inc., Princeton, NJ 08540-1437 (US)
(72) Inventor: The designation of the inventor has not yet been filed
(74) Representative: Gowshall, Jonathan Vallance

(57) **Abstract**

The present invention is directed to methods for the identification of a prostate cancer cell that has metastatic potential or a cell that is or is derived from a secondary prostate tumor metastasis by screening for the expression of flt-4, the cellular receptor of vascular endothelial growth factor-C and -D ("VEGF-C", "VEGF-D"). The present invention is also directed to methods for treating, inhibiting or preventing secondary prostate tumor metastases by inhibiting the expression or activity of flt-4,e.g., inhibiting flt-4: VEGF-C/D complex formation (binding), by administration of a therapeutic. Compositions useful in such methods are also provided.

SEQUENCE LISTING

<110> Sai L. Su

<120> METHODS FOR THE DIAGNOSIS AND TREATMENT OF METASTATIC
      PROSTATE TUMORS

<130> 20093-001000US

<140>
<141>

<150> PCT/US99/08079
<151> 1999-04-13

<160> 16

<170> PatentIn Ver. 2.0

<210> 1
<211> 4450
<212> DNA
<213> Homo sapiens

<220>
<221> CDS
<222> (22)..(3915)

<400> 1
acccacgcgc agcggccgga g atg cag cgg ggc gcc gcg ctg tgc ctg cga
   51
                        Met Gln Arg Gly Ala Ala Leu Cys Leu Arg
                          1               5                  10

ctg tgg ctc tgc ctg gga ctc ctg gac ggc ctg gtg agt gac tac tcc
   99
Leu Trp Leu Cys Leu Gly Leu Leu Asp Gly Leu Val Ser Asp Tyr Ser
                 15                  20                  25

atg acc ccc ccg acc ttg aac atc acg gag gag tca cac gtc atc gac
   147
Met Thr Pro Pro Thr Leu Asn Ile Thr Glu Glu Ser His Val Ile Asp
             30                  35                  40

acc ggt gac agc ctg tcc atc tcc tgc agg gga cag cac ccc ctc gag
   195
Thr Gly Asp Ser Leu Ser Ile Ser Cys Arg Gly Gln His Pro Leu Glu
         45                  50                  55

tgg gct tgg cca gga gct cag gag gcg cca gcc acc gga gac aag gac
   243

Trp Ala Trp Pro Gly Ala Gln Glu Ala Pro Ala Thr Gly Asp Lys Asp
    60                  65                  70

agc gag gac acg ggg gtg gtg cga gac tgc gag ggc aca gac gcc agg
291
Ser Glu Asp Thr Gly Val Val Arg Asp Cys Glu Gly Thr Asp Ala Arg
75                  80                  85                  90

ccc tac tgc aag gtg ttg ctg ctg cac gag gta cat gcc aac gac aca
339
Pro Tyr Cys Lys Val Leu Leu Leu His Glu Val His Ala Asn Asp Thr
                95                  100                 105

ggc agc tac gtc tgc tac tac aag tac atc aag gca cgc atc gag ggc
387
Gly Ser Tyr Val Cys Tyr Tyr Lys Tyr Ile Lys Ala Arg Ile Glu Gly
            110                 115                 120

acc acg gcc gcc agc tcc tac gtg ttc gtg aga gac ttt gag cag cca
435
Thr Thr Ala Ala Ser Ser Tyr Val Phe Val Arg Asp Phe Glu Gln Pro
        125                 130                 135

ttc atc aac aag cct gac acg ctc ttg gtc aac agg aag gac gcc atg
483
Phe Ile Asn Lys Pro Asp Thr Leu Leu Val Asn Arg Lys Asp Ala Met
    140                 145                 150

tgg gtg ccc tgt ctg gtg tcc atc ccc ggc ctc aat gtc acg ctg cgc
531
Trp Val Pro Cys Leu Val Ser Ile Pro Gly Leu Asn Val Thr Leu Arg
155                 160                 165                 170

tcg caa agc tcg gtg ctg tgg cca gac ggg cag gag gtg gtg tgg gat
579
Ser Gln Ser Ser Val Leu Trp Pro Asp Gly Gln Glu Val Val Trp Asp
                175                 180                 185

gac cgg cgg ggc atg ctc gtg tcc acg cca ctg ctg cac gat gcc ctg
627
Asp Arg Arg Gly Met Leu Val Ser Thr Pro Leu Leu His Asp Ala Leu
            190                 195                 200

tac ctg cag tgc gag acc acc tgg gga gac cag gac ttc ctt tcc aac
675
Tyr Leu Gln Cys Glu Thr Thr Trp Gly Asp Gln Asp Phe Leu Ser Asn
        205                 210                 215

ccc ttc ctg gtg cac atc aca ggc aac gag ctc tat gac atc cag ctg
723
Pro Phe Leu Val His Ile Thr Gly Asn Glu Leu Tyr Asp Ile Gln Leu
    220                 225                 230

ttg ccc agg aag tcg ctg gag ctg ctg gta ggg gag aag ctg gtc ctc
771
Leu Pro Arg Lys Ser Leu Glu Leu Leu Val Gly Glu Lys Leu Val Leu
235                 240                 245                 250

aac tgc acc gtg tgg gct gag ttt aac tca ggt gtc acc ttt gac tgg
819
Asn Cys Thr Val Trp Ala Glu Phe Asn Ser Gly Val Thr Phe Asp Trp
                255                 260                 265

gac tac cca ggg aag cag gca gag cgg ggt aag tgg gtg ccc gag cga
867
Asp Tyr Pro Gly Lys Gln Ala Glu Arg Gly Lys Trp Val Pro Glu Arg
            270                 275                 280

cgc tcc caa cag acc cac aca gaa ctc tcc agc atc ctg acc atc cac
915
Arg Ser Gln Gln Thr His Thr Glu Leu Ser Ser Ile Leu Thr Ile His
        285                 290                 295

aac gtc agc cag cac gac ctg ggc tcg tat gtg tgc aag gcc aac aac
963
Asn Val Ser Gln His Asp Leu Gly Ser Tyr Val Cys Lys Ala Asn Asn
    300                 305                 310

ggc atc cag cga ttt cgg gag agc acc gag gtc att gtg cat gaa aat
1011
Gly Ile Gln Arg Phe Arg Glu Ser Thr Glu Val Ile Val His Glu Asn
315                 320                 325                 330

ccc ttc atc agc gtc gag tgg ctc aaa gga ccc atc ctg gag gcc acg
1059
Pro Phe Ile Ser Val Glu Trp Leu Lys Gly Pro Ile Leu Glu Ala Thr
                335                 340                 345

gca gga gac gag ctg gtg aag ctg ccc gtg aag ctg gca gcg tac ccc
1107
Ala Gly Asp Glu Leu Val Lys Leu Pro Val Lys Leu Ala Ala Tyr Pro
            350                 355                 360

ccg ccc gag ttc cag tgg tac aag gat gga aag gca ctg tcc ggg cgc
1155
Pro Pro Glu Phe Gln Trp Tyr Lys Asp Gly Lys Ala Leu Ser Gly Arg
        365                 370                 375

cac agt cca cat gcc ctg gtg ctc aag gag gtg aca gag gcc agc aca
1203
His Ser Pro His Ala Leu Val Leu Lys Glu Val Thr Glu Ala Ser Thr
    380                 385                 390

ggc acc tac acc ctc gcc ctg tgg aac tcc gct gct ggc ctg agg cgc

1251
Gly Thr Tyr Thr Leu Ala Leu Trp Asn Ser Ala Ala Gly Leu Arg Arg
395                 400                 405                 410

aac atc agc ctg gag ctg gtg gtg aat gtg ccc ccc cag ata cat gag
   1299
Asn Ile Ser Leu Glu Leu Val Val Asn Val Pro Pro Gln Ile His Glu
                415                 420                 425

aag gag gcc tcc tcc ccc agc atc tac tcg cgt cac agc cgc cag gcc
   1347
Lys Glu Ala Ser Ser Pro Ser Ile Tyr Ser Arg His Ser Arg Gln Ala
            430                 435                 440

ctc acc tgc acg gcc tac ggg gtg ccc ctg cct ctc agc atc cag tgg
   1395
Leu Thr Cys Thr Ala Tyr Gly Val Pro Leu Pro Leu Ser Ile Gln Trp
        445                 450                 455

cac tgg cgg ccc tgg aca ccc tgc aag atg ttt gcc cag cgt agt ctc
   1443
His Trp Arg Pro Trp Thr Pro Cys Lys Met Phe Ala Gln Arg Ser Leu
    460                 465                 470

cgg cgg cgg cag cag caa gac ctc atg cca cag tgc cgt gac tgg agg
   1491
Arg Arg Arg Gln Gln Gln Asp Leu Met Pro Gln Cys Arg Asp Trp Arg
475                 480                 485                 490

gcg gtg acc acg cag gat gcc gtg aac ccc atc gag agc ctg gac acc
   1539
Ala Val Thr Thr Gln Asp Ala Val Asn Pro Ile Glu Ser Leu Asp Thr
                495                 500                 505

tgg acc gag ttt gtg gag gga aag aat aag act gtg agc aag ctg gtg
   1587
Trp Thr Glu Phe Val Glu Gly Lys Asn Lys Thr Val Ser Lys Leu Val
            510                 515                 520

atc cag aat gcc aac gtg tct gcc atg tac aag tgt gtg gtc tcc aac
   1635
Ile Gln Asn Ala Asn Val Ser Ala Met Tyr Lys Cys Val Val Ser Asn
        525                 530                 535

aag gtg ggc cag gat gag cgg ctc atc tac ttc tat gtg acc acc atc
   1683
Lys Val Gly Gln Asp Glu Arg Leu Ile Tyr Phe Tyr Val Thr Thr Ile
    540                 545                 550

ccc gac ggc ttc acc atc gaa tcc aag cca tcc gag gag cta cta gag
   1731
Pro Asp Gly Phe Thr Ile Glu Ser Lys Pro Ser Glu Glu Leu Leu Glu

555                 560                 565                 570

ggc cag ccg gtg ctc ctg agc tgc caa gcc gac agc tac aag tac gag
   1779
Gly Gln Pro Val Leu Leu Ser Cys Gln Ala Asp Ser Tyr Lys Tyr Glu
                575                 580                 585

cat ctg cgc tgg tac cgc ctc aac ctg tcc acg ctg cac gat gcg cac
   1827
His Leu Arg Trp Tyr Arg Leu Asn Leu Ser Thr Leu His Asp Ala His
            590                 595                 600

ggg aac ccg ctt ctg ctc gac tgc aag aac gtg cat ctg ttc gcc acc
   1875
Gly Asn Pro Leu Leu Leu Asp Cys Lys Asn Val His Leu Phe Ala Thr
        605                 610                 615

cct ctg gcc gcc agc ctg gag gag gtg gca cct ggg gcg cgc cac gcc
   1923
Pro Leu Ala Ala Ser Leu Glu Glu Val Ala Pro Gly Ala Arg His Ala
    620                 625                 630

acg ctc agc ctg agt atc ccc cgc gtc gcg ccc gag cac gag ggc cac
   1971
Thr Leu Ser Leu Ser Ile Pro Arg Val Ala Pro Glu His Glu Gly His
635                 640                 645                 650

tat gtg tgc gaa gtg caa gac cgg cgc agc cat gac aag cac tgc cac
   2019
Tyr Val Cys Glu Val Gln Asp Arg Arg Ser His Asp Lys His Cys His
                655                 660                 665

aag aag tac ctg tcg gtg cag gcc ctg gaa gcc cct cgg ctc acg cag
   2067
Lys Lys Tyr Leu Ser Val Gln Ala Leu Glu Ala Pro Arg Leu Thr Gln
            670                 675                 680

aac ttg acc gac ctc ctg gtg aac gtg agc gac tcg ctg gag atg cag
   2115
Asn Leu Thr Asp Leu Leu Val Asn Val Ser Asp Ser Leu Glu Met Gln
        685                 690                 695

tgc ttg gtg gcc gga gcg cac gcg ccc agc atc gtg tgg tac aaa gac
   2163
Cys Leu Val Ala Gly Ala His Ala Pro Ser Ile Val Trp Tyr Lys Asp
    700                 705                 710

gag agg ctg ctg gag gaa aag tct gga gtc gac ttg gcg gac tcc aac
   2211
Glu Arg Leu Leu Glu Glu Lys Ser Gly Val Asp Leu Ala Asp Ser Asn
715                 720                 725                 730

cag aag ctg agc atc cag cgc gtg cgc gag gag gat gcg gga ccg tat
2259
Gln Lys Leu Ser Ile Gln Arg Val Arg Glu Glu Asp Ala Gly Pro Tyr
                735                 740                 745

ctg tgc agc gtg tgc aga ccc aag ggc tgc gtc aac tcc tcc gcc agc
2307
Leu Cys Ser Val Cys Arg Pro Lys Gly Cys Val Asn Ser Ser Ala Ser
            750                 755                 760

gtg gcc gtg gaa ggc tcc gag gat aag ggc agc atg gag atc gtg atc
2355
Val Ala Val Glu Gly Ser Glu Asp Lys Gly Ser Met Glu Ile Val Ile
        765                 770                 775

ctt gtc ggt acc ggc gtc atc gct gtc ttc ttc tgg gtc ctc ctc ctc
2403
Leu Val Gly Thr Gly Val Ile Ala Val Phe Phe Trp Val Leu Leu Leu
    780                 785                 790

ctc atc ttc tgt aac atg agg agg ccg gcc cac gca gac atc aag acg
2451
Leu Ile Phe Cys Asn Met Arg Arg Pro Ala His Ala Asp Ile Lys Thr
795                 800                 805                 810

ggc tac ctg tcc atc atc atg gac ccc ggg gag gtg cct ctg gag gag
2499
Gly Tyr Leu Ser Ile Ile Met Asp Pro Gly Glu Val Pro Leu Glu Glu
                815                 820                 825

caa tgc gaa tac ctg tcc tac gat gcc agc cag tgg gaa ttc ccc cga
2547
Gln Cys Glu Tyr Leu Ser Tyr Asp Ala Ser Gln Trp Glu Phe Pro Arg
            830                 835                 840

gag cgg ctg cac ctg ggg aga gtg ctc ggc tac ggc gcc ttc ggg aag
2595
Glu Arg Leu His Leu Gly Arg Val Leu Gly Tyr Gly Ala Phe Gly Lys
        845                 850                 855

gtg gtg gaa gcc tcc gct ttc ggc atc cac aag ggc agc agc tgt gac
2643
Val Val Glu Ala Ser Ala Phe Gly Ile His Lys Gly Ser Ser Cys Asp
    860                 865                 870

acc gtg gcc gtg aaa atg ctg aaa gag ggc gcc acg gcc agc gag cag
2691
Thr Val Ala Val Lys Met Leu Lys Glu Gly Ala Thr Ala Ser Glu Gln
875                 880                 885                 890

cgc gcg ctg atg tcg gag ctc aag atc ctc att cac atc ggc aac cac
2739

Arg Ala Leu Met Ser Glu Leu Lys Ile Leu Ile His Ile Gly Asn His
                895                 900                 905

ctc aac gtg gtc aac ctc ctc ggg gcg tgc acc aag ccg cag ggc ccc
2787
Leu Asn Val Val Asn Leu Leu Gly Ala Cys Thr Lys Pro Gln Gly Pro
            910                 915                 920

ctc atg gtg atc gtg gag ttc tgc aag tac ggc aac ctc tcc aac ttc
2835
Leu Met Val Ile Val Glu Phe Cys Lys Tyr Gly Asn Leu Ser Asn Phe
        925                 930                 935

ctg cgc gcc aag cgg gac gcc ttc agc ccc tgc gcg gag aag tct ccc
2883
Leu Arg Ala Lys Arg Asp Ala Phe Ser Pro Cys Ala Glu Lys Ser Pro
    940                 945                 950

gag cag cgc gga cgc ttc cgc gcc atg gtg gag ctc gcc agg ctg gat
2931
Glu Gln Arg Gly Arg Phe Arg Ala Met Val Glu Leu Ala Arg Leu Asp
955                 960                 965                 970

cgg agg cgg ccg ggg agc agc gac agg gtc ctc ttc gcg cgg ttc tcg
2979
Arg Arg Arg Pro Gly Ser Ser Asp Arg Val Leu Phe Ala Arg Phe Ser
                975                 980                 985

aag acc gag ggc gga gcg agg cgg gct tct cca gac caa gaa gct gag
3027
Lys Thr Glu Gly Gly Ala Arg Arg Ala Ser Pro Asp Gln Glu Ala Glu
            990                 995                 1000

gac ctg tgg ctg agc ccg ctg acc atg gaa gat ctt gtc tgc tac agc
3075
Asp Leu Trp Leu Ser Pro Leu Thr Met Glu Asp Leu Val Cys Tyr Ser
        1005                1010                1015

ttc cag gtg gcc aga ggg atg gag ttc ctg gct tcc cga aag tgc atc
3123
Phe Gln Val Ala Arg Gly Met Glu Phe Leu Ala Ser Arg Lys Cys Ile
    1020                1025                1030

cac aga gac ctg gct gct cgg aac att ctg ctg tcg gaa agc gac gtg
3171
His Arg Asp Leu Ala Ala Arg Asn Ile Leu Leu Ser Glu Ser Asp Val
1035                1040                1045                1050

gtg aag atc tgt gac ttt ggc ctt gcc cgg gac atc tac aaa gac ccc
3219
Val Lys Ile Cys Asp Phe Gly Leu Ala Arg Asp Ile Tyr Lys Asp Pro
                1055                1060                1065

gac tac gtc cgc aag ggc agt gcc cgg ctg ccc ctg aag tgg atg gcc
3267
Asp Tyr Val Arg Lys Gly Ser Ala Arg Leu Pro Leu Lys Trp Met Ala
            1070                1075                1080

cct gaa agc atc ttc gac aag gtg tac acc acg cag agt gac gtg tgg
3315
Pro Glu Ser Ile Phe Asp Lys Val Tyr Thr Thr Gln Ser Asp Val Trp
        1085                1090                1095

tcc ttt ggg gtg ctt ctc tgg gag atc ttc tct ctg ggg gcc tcc ccg
3363
Ser Phe Gly Val Leu Leu Trp Glu Ile Phe Ser Leu Gly Ala Ser Pro
    1100                1105                1110

tac cct ggg gtg cag atc aat gag gag ttc tgc cag cgc gtg aga gac
3411
Tyr Pro Gly Val Gln Ile Asn Glu Glu Phe Cys Gln Arg Val Arg Asp
1115                1120                1125                1130

ggc aca agg atg agg gcc ccg gag ctg gcc act ccc gcc ata cgc cac
3459
Gly Thr Arg Met Arg Ala Pro Glu Leu Ala Thr Pro Ala Ile Arg His
                1135                1140                1145

atc atg ctg aac tgc tgg tcc gga gac ccc aag gcg aga cct gca ttc
3507
Ile Met Leu Asn Cys Trp Ser Gly Asp Pro Lys Ala Arg Pro Ala Phe
            1150                1155                1160

tcg gac ctg gtg gag atc ctg ggg gac ctg ctc cag ggc agg ggc ctg
3555
Ser Asp Leu Val Glu Ile Leu Gly Asp Leu Leu Gln Gly Arg Gly Leu
        1165                1170                1175

caa gag gaa gag gag gtc tgc atg gcc ccg cgc agc tct cag agc tca
3603
Gln Glu Glu Glu Glu Val Cys Met Ala Pro Arg Ser Ser Gln Ser Ser
    1180                1185                1190

gaa gag ggc agc ttc tcg cag gtg tcc acc atg gcc cta cac atc gcc
3651
Glu Glu Gly Ser Phe Ser Gln Val Ser Thr Met Ala Leu His Ile Ala
1195                1200                1205                1210

cag gct gac gct gag gac agc ccg cca agc ctg cag cgc cac agc ctg
3699
Gln Ala Asp Ala Glu Asp Ser Pro Pro Ser Leu Gln Arg His Ser Leu
                1215                1220                1225

gcc gcc agg tat tac aac tgg gtg tcc ttt ccc ggg tgc ctg gcc aga

3747
Ala Ala Arg Tyr Tyr Asn Trp Val Ser Phe Pro Gly Cys Leu Ala Arg
            1230                1235                1240

ggg gct gag acc cgt ggt tcc tcc agg atg aag aca ttt gag gaa ttc
   3795
Gly Ala Glu Thr Arg Gly Ser Ser Arg Met Lys Thr Phe Glu Glu Phe
        1245                1250                1255

ccc atg acc cca acg acc tac aaa ggc tct gtg gac aac cag aca gac
   3843
Pro Met Thr Pro Thr Thr Tyr Lys Gly Ser Val Asp Asn Gln Thr Asp
   1260                1265                1270

agt ggg atg gtg ctg gcc tcg gag gag ttt gag cag ata gag agc agg
   3891
Ser Gly Met Val Leu Ala Ser Glu Glu Phe Glu Gln Ile Glu Ser Arg
1275                1280                1285                1290

cat aga caa gaa agc ggc ttc agg tagctgaagc agagagagag aaggcagca
t  3945
His Arg Gln Glu Ser Gly Phe Arg

                1295

acgtcagcat tttcttctct gcacttataa gaaagatcaa agactttaag actttcgc
ta 4005

tttcttctac tgctatctac tacaaacttc aaagaggaac caggaggaca agaggagc
at 4065

gaaagtggac aaggagtgtg accactgaag caccacaggg aggggttagg cctccgga
tg 4125

actgcgggca ggcctggata atatccagcc tcccacaaga agctggtgga gcagagtg
tt 4185

ccctgactcc tccaaggaaa gggagacgcc ctttcatggt ctgctgagta acaggtgc
ct 4245

tcccagacac tggcgttact gcttgaccaa agagccctca agcggccctt atgccagc
gt 4305

gacagagggc tcacctcttg ccttctaggt cacttctcac aatgtccctt cagcacct
ga 4365

ccctgtgccc gccgattatt ccttggtaat atgagtaata catcaaagag tagtatta
aa 4425

agctaattaa tcatgtttat aaaaa
   4450

<210> 2
<211> 1298
<212> PRT
<213> Homo sapiens

<400> 2
Met Gln Arg Gly Ala Ala Leu Cys Leu Arg Leu Trp Leu Cys Leu Gly
1               5                   10                  15

Leu Leu Asp Gly Leu Val Ser Asp Tyr Ser Met Thr Pro Pro Thr Leu
            20                  25                  30

Asn Ile Thr Glu Glu Ser His Val Ile Asp Thr Gly Asp Ser Leu Ser
        35                  40                  45

Ile Ser Cys Arg Gly Gln His Pro Leu Glu Trp Ala Trp Pro Gly Ala
    50                  55                  60

Gln Glu Ala Pro Ala Thr Gly Asp Lys Asp Ser Glu Asp Thr Gly Val
65                  70                  75                  80

Val Arg Asp Cys Glu Gly Thr Asp Ala Arg Pro Tyr Cys Lys Val Leu
                85                  90                  95

Leu Leu His Glu Val His Ala Asn Asp Thr Gly Ser Tyr Val Cys Tyr
            100                 105                 110

Tyr Lys Tyr Ile Lys Ala Arg Ile Glu Gly Thr Thr Ala Ala Ser Ser
        115                 120                 125

Tyr Val Phe Val Arg Asp Phe Glu Gln Pro Phe Ile Asn Lys Pro Asp
    130                 135                 140

Thr Leu Leu Val Asn Arg Lys Asp Ala Met Trp Val Pro Cys Leu Val
145                 150                 155                 160

Ser Ile Pro Gly Leu Asn Val Thr Leu Arg Ser Gln Ser Ser Val Leu
                165                 170                 175

Trp Pro Asp Gly Gln Glu Val Val Trp Asp Asp Arg Arg Gly Met Leu
            180                 185                 190

Val Ser Thr Pro Leu Leu His Asp Ala Leu Tyr Leu Gln Cys Glu Thr
        195                 200                 205

Thr Trp Gly Asp Gln Asp Phe Leu Ser Asn Pro Phe Leu Val His Ile
    210                 215                 220

Thr Gly Asn Glu Leu Tyr Asp Ile Gln Leu Leu Pro Arg Lys Ser Leu
225                 230                 235                 240

Glu Leu Leu Val Gly Glu Lys Leu Val Leu Asn Cys Thr Val Trp Ala
                245                 250                 255

Glu Phe Asn Ser Gly Val Thr Phe Asp Trp Asp Tyr Pro Gly Lys Gln
            260                 265                 270

Ala Glu Arg Gly Lys Trp Val Pro Glu Arg Arg Ser Gln Gln Thr His
        275                 280                 285

Thr Glu Leu Ser Ser Ile Leu Thr Ile His Asn Val Ser Gln His Asp
    290                 295                 300

Leu Gly Ser Tyr Val Cys Lys Ala Asn Asn Gly Ile Gln Arg Phe Arg

305                 310                 315                 320

Glu Ser Thr Glu Val Ile Val His Glu Asn Pro Phe Ile Ser Val Glu

                325                 330                 335

Trp Leu Lys Gly Pro Ile Leu Glu Ala Thr Ala Gly Asp Glu Leu Val

            340                 345                 350

Lys Leu Pro Val Lys Leu Ala Ala Tyr Pro Pro Pro Glu Phe Gln Trp

        355                 360                 365

Tyr Lys Asp Gly Lys Ala Leu Ser Gly Arg His Ser Pro His Ala Leu

    370                 375                 380

Val Leu Lys Glu Val Thr Glu Ala Ser Thr Gly Thr Tyr Thr Leu Ala

385                 390                 395                 400

Leu Trp Asn Ser Ala Ala Gly Leu Arg Arg Asn Ile Ser Leu Glu Leu

                405                 410                 415

Val Val Asn Val Pro Pro Gln Ile His Glu Lys Glu Ala Ser Ser Pro

            420                 425                 430

Ser Ile Tyr Ser Arg His Ser Arg Gln Ala Leu Thr Cys Thr Ala Tyr

        435                 440                 445

Gly Val Pro Leu Pro Leu Ser Ile Gln Trp His Trp Arg Pro Trp Thr

    450                 455                 460

Pro Cys Lys Met Phe Ala Gln Arg Ser Leu Arg Arg Arg Gln Gln Gln

465                 470                 475                 480

Asp Leu Met Pro Gln Cys Arg Asp Trp Arg Ala Val Thr Thr Gln Asp
                485                 490                 495

Ala Val Asn Pro Ile Glu Ser Leu Asp Thr Trp Thr Glu Phe Val Glu
            500                 505                 510

Gly Lys Asn Lys Thr Val Ser Lys Leu Val Ile Gln Asn Ala Asn Val
        515                 520                 525

Ser Ala Met Tyr Lys Cys Val Val Ser Asn Lys Val Gly Gln Asp Glu
    530                 535                 540

Arg Leu Ile Tyr Phe Tyr Val Thr Thr Ile Pro Asp Gly Phe Thr Ile
545                 550                 555                 560

Glu Ser Lys Pro Ser Glu Glu Leu Leu Glu Gly Gln Pro Val Leu Leu
                565                 570                 575

Ser Cys Gln Ala Asp Ser Tyr Lys Tyr Glu His Leu Arg Trp Tyr Arg
            580                 585                 590

Leu Asn Leu Ser Thr Leu His Asp Ala His Gly Asn Pro Leu Leu Leu
        595                 600                 605

Asp Cys Lys Asn Val His Leu Phe Ala Thr Pro Leu Ala Ala Ser Leu
    610                 615                 620

Glu Glu Val Ala Pro Gly Ala Arg His Ala Thr Leu Ser Leu Ser Ile
625                 630                 635                 640

Pro Arg Val Ala Pro Glu His Glu Gly His Tyr Val Cys Glu Val Gln
                645                 650                 655

Asp Arg Arg Ser His Asp Lys His Cys His Lys Lys Tyr Leu Ser Val
            660                 665                 670

Gln Ala Leu Glu Ala Pro Arg Leu Thr Gln Asn Leu Thr Asp Leu Leu
        675                 680                 685

Val Asn Val Ser Asp Ser Leu Glu Met Gln Cys Leu Val Ala Gly Ala
    690                 695                 700

His Ala Pro Ser Ile Val Trp Tyr Lys Asp Glu Arg Leu Leu Glu Glu
705                 710                 715                 720

Lys Ser Gly Val Asp Leu Ala Asp Ser Asn Gln Lys Leu Ser Ile Gln
                725                 730                 735

Arg Val Arg Glu Glu Asp Ala Gly Pro Tyr Leu Cys Ser Val Cys Arg
            740                 745                 750

Pro Lys Gly Cys Val Asn Ser Ser Ala Ser Val Ala Val Glu Gly Ser
        755                 760                 765

Glu Asp Lys Gly Ser Met Glu Ile Val Ile Leu Val Gly Thr Gly Val
    770                 775                 780

Ile Ala Val Phe Phe Trp Val Leu Leu Leu Leu Ile Phe Cys Asn Met
785                 790                 795                 800

Arg Arg Pro Ala His Ala Asp Ile Lys Thr Gly Tyr Leu Ser Ile Ile

                805                 810                 815

Met Asp Pro Gly Glu Val Pro Leu Glu Glu Gln Cys Glu Tyr Leu Ser

            820                 825                 830

Tyr Asp Ala Ser Gln Trp Glu Phe Pro Arg Glu Arg Leu His Leu Gly

        835                 840                 845

Arg Val Leu Gly Tyr Gly Ala Phe Gly Lys Val Val Glu Ala Ser Ala

    850                 855                 860

Phe Gly Ile His Lys Gly Ser Ser Cys Asp Thr Val Ala Val Lys Met

865                 870                 875                 880

Leu Lys Glu Gly Ala Thr Ala Ser Glu Gln Arg Ala Leu Met Ser Glu

                885                 890                 895

Leu Lys Ile Leu Ile His Ile Gly Asn His Leu Asn Val Val Asn Leu

            900                 905                 910

Leu Gly Ala Cys Thr Lys Pro Gln Gly Pro Leu Met Val Ile Val Glu

        915                 920                 925

Phe Cys Lys Tyr Gly Asn Leu Ser Asn Phe Leu Arg Ala Lys Arg Asp

    930                 935                 940

Ala Phe Ser Pro Cys Ala Glu Lys Ser Pro Glu Gln Arg Gly Arg Phe

945                 950                 955                 960

Arg Ala Met Val Glu Leu Ala Arg Leu Asp Arg Arg Arg Pro Gly Ser

                965                 970                 975

Ser Asp Arg Val Leu Phe Ala Arg Phe Ser Lys Thr Glu Gly Gly Ala
            980                 985                 990
Arg Arg Ala Ser Pro Asp Gln Glu Ala Glu Asp Leu Trp Leu Ser Pro
        995                 1000                1005
Leu Thr Met Glu Asp Leu Val Cys Tyr Ser Phe Gln Val Ala Arg Gly
    1010                1015                1020
Met Glu Phe Leu Ala Ser Arg Lys Cys Ile His Arg Asp Leu Ala Ala
025 1030 1035 1040
Arg Asn Ile Leu Leu Ser Glu Ser Asp Val Val Lys Ile Cys Asp Phe
                1045                1050                1055
Gly Leu Ala Arg Asp Ile Tyr Lys Asp Pro Asp Tyr Val Arg Lys Gly
            1060                1065                1070
Ser Ala Arg Leu Pro Leu Lys Trp Met Ala Pro Glu Ser Ile Phe Asp
        1075                1080                1085
Lys Val Tyr Thr Thr Gln Ser Asp Val Trp Ser Phe Gly Val Leu Leu
    1090                1095                1100
Trp Glu Ile Phe Ser Leu Gly Ala Ser Pro Tyr Pro Gly Val Gln Ile
105 1110 1115 1120
Asn Glu Glu Phe Cys Gln Arg Val Arg Asp Gly Thr Arg Met Arg Ala
                1125                1130                1135
Pro Glu Leu Ala Thr Pro Ala Ile Arg His Ile Met Leu Asn Cys Trp

            1140                1145                1150

Ser Gly Asp Pro Lys Ala Arg Pro Ala Phe Ser Asp Leu Val Glu Ile

        1155                1160                1165

Leu Gly Asp Leu Leu Gln Gly Arg Gly Leu Gln Glu Glu Glu Glu Val

    1170                1175                1180

Cys Met Ala Pro Arg Ser Ser Gln Ser Ser Glu Glu Gly Ser Phe Ser

185 1190 1195 1200

Gln Val Ser Thr Met Ala Leu His Ile Ala Gln Ala Asp Ala Glu Asp

                1205                1210                1215

Ser Pro Pro Ser Leu Gln Arg His Ser Leu Ala Ala Arg Tyr Tyr Asn

            1220                1225                1230

Trp Val Ser Phe Pro Gly Cys Leu Ala Arg Gly Ala Glu Thr Arg Gly

        1235                1240                1245

Ser Ser Arg Met Lys Thr Phe Glu Glu Phe Pro Met Thr Pro Thr Thr

    1250                1255                1260

Tyr Lys Gly Ser Val Asp Asn Gln Thr Asp Ser Gly Met Val Leu Ala

265 1270 1275 1280

Ser Glu Glu Phe Glu Gln Ile Glu Ser Arg His Arg Gln Glu Ser Gly

                1285                1290                1295

Phe Arg

<210> 3
<211> 15
<212> DNA
<213> Artificial Sequence

<220>
<223> Description of Artificial Sequence:
      oligonucleotide

<400> 3
ggcgccccgc tgcat
   15


<210> 4
<211> 23
<212> DNA
<213> Artificial Sequence

<220>
<223> Description of Artificial Sequence: primer

<400> 4
cgaagtggtg aagttcatgg atg
   23


<210> 5
<211> 25
<212> DNA
<213> Artificial Sequence

<220>
<223> Description of Artificial Sequence: primer

<400> 5
ttctgtatca gtctttcctg gtgag
   25


<210> 6
<211> 24
<212> DNA
<213> Artificial Sequence

<220>
<223> Description of Artificial Sequence: primer

<400> 6

ctggcatggt cttctgtgaa agca
24

<210> 7
<211> 22
<212> DNA
<213> Artificial Sequence

<220>
<223> Description of Artificial Sequence: primer

<400> 7
aataccagtg gatgtgatgc gg
22

<210> 8
<211> 20
<212> DNA
<213> Artificial Sequence

<220>
<223> Description of Artificial Sequence: primer

<400> 8
taccacagtg tcaggcagcg
20

<210> 9
<211> 20
<212> DNA
<213> Artificial Sequence

<220>
<223> Description of Artificial Sequence: primer

<400> 9
atcaaattct cggttggccc
20

<210> 10
<211> 20
<212> DNA
<213> Artificial Sequence

<220>
<223> Description of Artificial Sequence: primer

<400> 10

agagggatgg agttcctggc
   20


<210> 11
<211> 22
<212> DNA
<213> Artificial Sequence

<220>
<223> Description of Artificial Sequence: primer

<400> 11
aataccagtg gatgtgatgc gg
   22


<210> 12
<211> 21
<212> DNA
<213> Artificial Sequence

<220>
<223> Description of Artificial Sequence:
      oligonucleotide

<400> 12
agacagcaga aagttcatgg t
   21


<210> 13
<211> 15
<212> DNA
<213> Artificial Sequence

<220>
<223> Description of Artificial Sequence:
      oligonucleotide

<400> 13
caagtgcatg gtgga
   15


<210> 14
<211> 15
<212> DNA
<213> Artificial Sequence

<220>
<223> Description of Artificial Sequence:

oligonucleotide

<400> 14
caccttgctc tgcat
15

<210> 15
<211> 14
<212> DNA
<213> Artificial Sequence

<220>
<223> Description of Artificial Sequence:
oligonucleotide

<400> 15
cccggtgtcc caga
14

<210> 16
<211> 17
<212> DNA
<213> Artificial Sequence

<220>
<223> Description of Artificial Sequence:
oligonucleotide

<400> 16
tacgtagtat ggtgtac
17

## Description

### 1. FIELD OF THE INVENTION

The present invention is directed to methods for the identification of a prostate cancer cell that has metastatic potential or a cell that is a secondary prostate tumor metastasis, or is derived therefrom, by screening for the expression or activity of flt-4, the cellular receptor of vascular endothelial growth factor-C ("VEGF-C") and -D ("VEGF-D"). The present invention is also directed to methods for treating, inhibiting or preventing secondary prostate tumor metastases by inhibiting the expression or activity of flt-4, *e.g.*, inhibiting flt-4:VEGF-C complex or flt-4-VEGF-D complex formation (binding). Compositions useful in such methods are also provided.

### 2. BACKGROUND OF THE INVENTION

The development and progression of prostate cancer remains poorly understood. Neoplastic transformation involves multiple mechanisms, such as p53 or ras mutations, and/or imbalance in growth regulatory factors.

VEGF is a heparin-binding, dimeric polypeptide growth factor originally purified based on its vascular permeability- enhancing activity and subsequently shown to be a potent mitogen for endothelial cells (reviewed in Shibuya, 1995, Adv. Cancer Res. 67:281-316). VEGF influences both angiogenesis and vascular permeability in solid tumors (Ferrara, 1995, Breast Cancer Res. Treat. 36:127-137; Ferrara et al., 1991, J. Cell Biochem. 47:211-8.; Klagsbrun et al., 1996, Cytokine Growth Factor Rev. 7:259-70). It is also produced by some normal tissues, particularly during the menstrual cycle (Shweiki et al., 1993, J. Clin. Invest. 91:2235-2243) and wound healing (Frank et al., 1995, J. Biol. Chem. 270:12607-12613), and is thought to play important role(s) in the angiogenic pathology of diabetic retinopathy and rheumatoid disorders (Ferrara, 1995, Breast Cancer Res. Treat. 36:127-137).

In the prostate, the expression of VEGF has been reported both in non-malignant epithelium (Brown et al., 1995, J. Urol. 154:576-579; Jackson et al., 1997, J. Urol. 157:2323-2328) as well as in cancer cells (Ferrer et al., 1997, J. Urol. 157:2329-2333; Ferrer et al., 1998, Urology 51:161-167; Harper et al., 1996, Br. J. Cancer 74:910-916; Jackson et al., 1997, J. Urol. 157:2323-2328). Localization studies showed the presence of VEGF, at both mRNA and protein levels, in prostate cancer cells, tumor-associated stroma, and a proportion of benign prostate hyperplasia ("BPH") epithelial cells (Jackson et al., 1997, J. Urol. 157:2323-2328). The relative level of VEGF in normal and transformed prostate epithelium remains controversial and unclear (Woessner et al., 1998, Exp. Mol. Pathol. 65:37-52; Chevalier, 1997, J. Urol. 157:2040-2041). In prostate cancer, increased microvessel density was found to correlate with disease stage and metastasis (Bigler et al., 1993, Hum. Pathol. 24:220-6; Brawer et al., 1994, Cancer 73:678-687.; Deering et al., 1995, Prostate 26:111-115.; Siegal et al., 1995, Cancer 75:2545-2451; Weidner et al., 1996, Important Adv. Oncol. 167-190.). Increased neo-vascularization is also found in prostatic intraepithelial neoplasia ("PIN") (Brawer et al., 1994, Cancer 73:678-687; Ferrer et al., 1997, J. Urol. 157:2329-2333; Ferrer et al., 1998, Urology 51:161-167; Harper et al., 1996, Br. J. Cancer 74:910-916) and latent carcinoma (Furusato et al., 1994, Br. J. Cancer 70:1244-1246). Elevated levels of angiogenesis are found in human cell lines PC-3 and DU-145-derived tumors when implanted in nude mice (Connolly et al., 1998, J. Urol. 160:932-936). Taken together, these studies suggest a trophic role for VEGF in supporting tumor growth via angiogenesis.

VEGF-C is another endothelial growth factor with 32% amino acid identity to VEGF and was originally cloned from a human prostate hormone refractory cell line, PC-3 (Joukov et al., 1997, J. Cell Physiol. 173:211-215; Joukov et al., 1996, EMBO J., 15:290-298; Joukov et al., 1997, EMBO J. 16:3898-3911). Whereas VEGF is specific to endothelial cells of blood vessels, VEGF-C is a lymphangiogenic factor. The receptors for VEGF-C, both flk-1 and flt-4, are expressed in lymphatic endothelial cells and melanocytes (Kaipainen et al., 1993, J. Exp. Med. 178:2077-2088). Experiments with transgenic mice showed that the expression of VEGF-C is associated with the development of lymphatic vessels in normal tissues (Jeltsch et al., 1997, Science 276:1423-1425). VEGF-C induced growth of lymphatic vessels in chick chorioallantoic membrane (Oh et al., 1997, Dev. Biol. 188:96-109).

Flt-4 is a receptor type tyrosine kinase (RTK) with 7 Ig-like domains similar to other VEGF receptors. Expression of flt-4 was initially localized to angioblasts and venules in the early embryo. It becomes restricted to lymphatic endothelial cells during later development, and some high endothelial venules in human adult tissues (Kaipainen et al., 1995, Proc. Natl. Acad. Sci. USA 92:3566-3570; Kaipainen et al., 1993, J. Exp. Med. 178: 2077-2088). Expression of Flt-4 is consistent with known lymphatic vascular pattern in human skin (Lymboussaki et al., 1998, Am. J. Pathol. 153: 395-403). This expression pattern suggested that Flt-4 may play a role in the regulation of lymphangioenesis (Kaipainen et al., 1995, Proc. Natl. Acad. Sci. USA 92:3566-3570; Kaipainen et al., 1993, J Exp Med 178: 2077-2088; Mustonen et al., 1995, J. Cell Biol. 129: 895-898). Expression of Flt-4 has also been found in melanocytes (Gitay-Goren et al., 1993, Biochem. Biophys. Res. Commun. 190:702-708). In addition to VEGF-C, VEGF-D is also a ligand for flt-4 (Achen et al., 1998, Proc. Natl. Acad. Sci. USA 95:548-553; Yamada et al., 1997, Genomics 42:483-488).

Citation or identification of any reference in Section 2 or any other section of this application shall not be construed as an admission that such reference is available as prior art to the present invention.

### 3. SUMMARY OF THE INVENTION

The present invention is based, in part, on the surprising discovery that the expression of flt-4 in a prostate cell indicates that such prostate cell is a cancerous prostate cell that has metastatic potential or is a secondary tumor metastasis of a primary prostate tumor, or is derived therefrom.

The present invention is directed to methods for detection of metastatic potential comprising detecting expression or activity of flt-4 in a prostate cancer cell, wherein expression or activity of flt-4 indicates that said cell has metastatic potential. The present invention is also directed to methods for detection of metastatic potential comprising identifying a prostate cell in a body fluid sample obtained from a subject and detecting expression or activity of flt-4 in said cells, wherein expression of flt-4 indicates that said cell is a prostate cancer cell that has metastatic potential or is a secondary tumor metastasis or is derived therefrom. The prostate cell can be identified using a prostate cell-specific marker and can be obtained from a body fluid sample, *e.g.*, blood, urine, semen. In a preferred embodiment, the prostate cell can be identified and screened for flt-4 expression simultaneously using an antibody specific for a prostate cell-specific marker and an antibody specific for flt-4 in a flow cytometer. In another preferred embodiment, the prostate cell can be identified and screened for flt-4 expression simultaneously using an antibody specific for a prostate cell-specific marker and an antibody specific for flt-4 using a laser scanning cytometer.

The present invention is also directed to methods for diagnosing metastatic prostate cancer in a subject comprising identifying a prostate cell in a body fluid sample obtained from the subject and detecting expression or activity of flt-4 in the prostate cell, wherein expression or activity of flt-4 in said cell indicates that the subject has metastatic prostate cancer. The present invention is also directed to methods for determining the prognosis of a subject with prostate cancer comprising identifying a prostate cell in a body fluid sample obtained from a subject with prostate cancer and detecting expression or activity of flt-4 in the prostate cell, wherein expression or activity of flt-4 in the prostate cell obtained from the subject indicates that the subject has a worse prognosis as compared to a second subject in whose prostate cell no flt-4 expression or activity is detected. Methods for monitoring the efficacy of a method of treatment or of inhibition of metastatic prostate cancer are also provided.

The present invention is directed to methods for treating, inhibiting or preventing a secondary prostate tumor metastasis in a subject by administering one or more compounds or molecules that inhibits flt-4 expression or activity. Illustrative examples of such compounds or molecules include, but are not limited to, fragments of flt-4 or nucleic acid molecules encoding the same, antisense oligonucleotides to inhibit expression of flt-4, antibodies specific to flt-4 or to a complex of flt-4 and its ligand VEGF-C or its ligand VEGF-D, etc. The present invention is further directed to compositions which can be used in the above described methods.

The present invention is further directed to methods for inhibiting the activity of a flt-4:VEGF-C complex or flt-4:VEGF-D complex. Methods that identify molecules that inhibit flt-4 expression or activity are also provided. Animal models and methods for screening for modulators (*i.e.*, agonists and antagonists) of the expression or activity of flt-4 or the activity of a flt-4:VEGF-C or flt-4:VEGF-D complex are also provided.

### 4. BRIEF DESCRIPTION OF THE FIGURES

Figs. 1A-1F set forth the nucleotide sequence (SEQ ID NO:1) and the amino acid sequence (SEQ ID NO:2), respectively, of the human flt-4 gene.
Figs. 2A-2F are photographs of anti-flt-4 antibody-stained prostatic tissue sections. Fig. 2A: normal lymph node; Fig. 2B: benign prostatic hyperplasia tissue; Fig. 2C, 2D: lymph node with prostatic metastases; Fig. 2E: benign prostatic hyperplasia tissue and prostate cancer; and Fig. 2F: prostate cancer.
Figs. 3A-3D are photographs of anti-flt-4 antibody stained human prostate cell lines. Fig. 3A: LNCaP cell line; Fig. 3B: PC3 cell line; Fig. 3C: DU145; and Fig. 3D: TSUPr1 cell line.
Fig. 4 is a photograph of agarose gels showing the results of reverse transcriptase polymerase chain reactions ("RT-PCR") testing for expression of VEGF, VEGF-C and their receptors flk-1 and flt-4, respectively, in several human prostate cancer cell lines.
Fig. 5 is a graph showing that flt-4 expression can be detected on the surface of live LNCaP cells using an anti-flt-4 antibody.
Fig. 6 is a histogram showing the results of antisense oligonucleotide treatment of a human prostate cancer cell line.

### 5. DETAILED DESCRIPTION OF THE INVENTION

The present invention is based, in part, on the surprising discovery that the expression of flt-4 in a prostate cell indicates that such prostate cell is a cancerous prostate cell that has metastatic potential or is a secondary tumor metastasis of a primary prostate tumor, or is derived therefrom. Not intending to be limited to a particular mechanism of action, although it was known that VEGF-C and its cell surface receptor flt-4 are implicated in the promotion of lymphoangiogenesis, prior to the present invention, it was not known that VEGF-C and flt-4 are involved in an autocrine feedback loop resulting in the promotion and formation of secondary prostate tumor metastases. Further, the present inventor believes that VEGF-D, another ligand of flt-4, and flt-4 are also involved in an autocrine feedback loop resulting in the promotion and formation of secondary prostate tumor metastases.

The present invention is directed to methods for detection of metastatic potential comprising detecting expression or activity of flt-4 in a prostate cancer cell, wherein expression or activity of flt-4 indicates that said cell has metastatic potential. The present invention is also directed to methods for detection of metastatic potential comprising identifying a prostate cell in a body fluid sample obtained from a subject and detecting expression or activity of flt-4 in said cells, wherein expression of flt-4 indicates that said cell is a prostate cancer cell that has metastatic potential or is a secondary tumor metastasis, or is derived therefrom. The prostate cell can be identified using a prostate cell-specific marker and can be obtained from a body fluid sample, *e.g.*, blood, urine, semen. In a preferred embodiment, the prostate cell can be identified and screened for flt-4 expression simultaneously using an antibody specific for a prostate cell-specific marker and an antibody specific for flt-4 in a flow cytometer.

Methods for treating, inhibiting or preventing secondary prostate tumor metastases by administration to a subject in need of such treatment, inhibition or prevention by administration of a molecule that inhibits the expression or activity of flt-4 are provided. Examples of such molecules include, but are not limited to, a fragment of flt-4 that acts as a competitive inhibitor of flt-4 binding to its ligands VEGF-C or VEGF-D, antibodies to flt-4, antisense nucleic acids, etc.. Methods for the diagnosis, prognosis and screening for secondary prostate tumor metastases are also provided. Compositions for carrying out such methods are also provided in the present invention. For example, such methods can be used by a pathologist screening tissue biopsies, such as prostate tissue or lymph node tissue biopsies, for a prostate cell with metastatic potential.

The present invention is also directed to methods of assaying for the presence of a flt-4:VEGF-C complex or a flt-4:VEGF-D complex ("flt-4:VEGF-C/D") for diagnosis and/or prognosis of prostate cancer. As used in the present application "flt-4:VEGF-C/D" indicates a complex of flt-4 and VEGF-C or a complex of flt-4 and VEGF-D.

For clarity of disclosure, and not by way of limitation, a detailed description of the invention is divided into the following subsections.

### 5.1. FLT-4 EXPRESSION IN PROSTATE CELLS

Flt-4 expression in a prostate cancer cell indicates that the prostate cancer cell has metastatic potential or indicates that the prostate cancer cell is a secondary prostate tumor metastasis, or is derived therefrom, and thus flt-4 expression has diagnostic and prognostic utility in assessing the metastatic potential of a prostate cancer cell or in screening for the presence of secondary tumor metastases derived from a primary prostate tumor.

In one embodiment of the invention, prostate cells are identified by expression of a prostate cell-specific marker and the identified prostate cells are screened for flt-4 expression. In another embodiment of the invention, the prostate cells are identified and screened concurrently with labeled antibodies specific for a prostate cell-specific marker and specific for flt-4, respectively, using a flow cytometer.

Detecting levels of flt-4:VEGF-C/D complexes, or flt-4 protein expression, or detecting the level of mRNAs encoding flt-4 in a prostate cell, may be used to determine whether the prostate cell has metastatic potential or whether the prostate cell is a secondary prostate tumor metastasis, or is derived therefrom. Moreover, the detection of flt-4:VEGF-C/D complexes or flt-4 expression in a prostate cell can be used in the prognosis of prostate cancer, to follow the course of prostate cancer, to follow a therapeutic response, etc.

Prostate cells can be detected by any method known to those of skill in the art. For example, prostate cells can be detected using an antibody specific for a prostate cell-specific marker or using a fragment comprising the binding domain of an antibody specific for a prostate cell marker. Preferably, the antibodies used are monoclonal antibodies.

Prostate cell-specific markers are known and include prostate-specific antigen (PSA), prostate-specific membrane antigen (PSMA), prostate secretory protein (PSP), prostate acid phosphatase (PAP), and human glandular kallekrein 2 (HK-2). Another prostate cell-specific marker is prostate stem cell antigen (PSCA) identified by Reiter et al., 1998, Proc. Natl. Acad. Sci. USA 95:1735-1740. Yet another prostate cell-specific marker is PTI-1, a prostate carcinoma oncogene identified by Shen et al., 1995, Proc. Natl. Acad. Sci. USA 92:6778-6782. For a general review of prostate cell-specific markers, see Nelson et al., 1998, Genomics 47:12-25.

In a preferred embodiment, the prostate cells are detected by using a monoclonal antibody specific for PSMA. PSMA is an approximately 120 kDa molecular weight protein expressed in prostate tissues and was originally identified by reactivity with a monoclonal antibody designated 7E11-C5. Horoszewicz et al., 1987, Anticancer Res. 7:927-935; U.S. Patent No. 5,162,504. PSMA was obtained in purified form (Wright et al., 1990, *Antibody Immunoconjugates and Radio Pharmaceuticals* 3:Abstract 193) and characterized as a type II transmembrane protein having some sequence identity with the transferrin receptor (Israeli et al., 1994, Cancer Res. 54:1807-1811) and with NAALADase activity (Carter et al., 1996, Proc. Natl. Acad. Sci. USA 93:749-753). More importantly, although PSMA is expressed in normal prostate, benign prostate hyperplasia and prostate cancer, PSMA is expressed in increased amounts in prostate cancer, and an elevated level of PSMA is also detectable in the sera of these prostate cancer patients. Horoszewicz et al., 1987, *supra*; Rochon et al., 1994, Prostate 25:219-223; Murphy et al., 1995, Prostate 26:164-168; and Murphy et al., 1995, Anticancer Res. 15:1473-1479. A cDNA encoding PSMA has been cloned. Israeli et al., 1993, Cancer Res. 53:227-230.

PSMA-specific antibodies are known and include 7E11.C5 (ATCC Accession No. HB10494); 3F5.4G6 (ATCC Accession No. HB12060); 3D7-1.1 (ATCC Accession No. HB12309); 4E10-1.14 (ATCC Accession No. HB12310); 1G3 (ATCC Accession No. HB-12489); 1G9 (ATCC Accession No. HB-12495); 2C7 (ATCC Accession No. HB-12490); 3C4 (ATCC Accession No. HB-12494); 3C6 (ATCC Accession No. HB-12491); 3C9 (ATCC Accession No. HB-12484); 3E6 (ATCC Accession No. HB-12486); 3E11 (ATCC Accession No. HB-12488); 3G6 (ATCC Accession No. 12485); 4D4 (ATCC Accession No. HB-12493); 4D8 (ATCC Accession No. HB-12487); or 4C8B9 (ATCC Accession No. HB-12492), see WO 97/35616. Other PSMA-specific antibodies which can be employed in the present invention include E99 (ATCC Accession No. HB-12101); J415 (ATCC Accession No. HB-12109); J533 (ATCC Accession No. HB-12127); and J591 (ATCC Accession No. HB-12126), each isolated by Bander, International Patent Publication WO 98/03873. Additional non-limiting examples of antibodies specific for PSMA, which can be used in the methods of the present invention, are presented in Table I. All the antibodies presented in Table I are murine IgG monoclonal antibodies, which are reactive to native PSMA. The approximate location of the binding epitope of each antibody, as well as the isotype subclass of each antibody, is also summarized in Table I.

**TABLE I**

| Binding Specificity and Isotype of PSMA-Specific Antibodies to Native PSMA and PSMA Fragments | | | | | |
|---|---|---|---|---|---|
| **Antibody** | **Native PSMA** | **1-173** | **134-437** | **437-750** | **Isotype**^{**a**} |
| 3F6 | + | - | - | - | IgG_{2b} |
| 2E4 | + | weak | - | - | IgG₂ₐ |
| 3C2 | + | + | + | - | IgG₂ₐ |
| 4C8G8 | + | - | + | - | IgG_{2b} |
| 2C4 | + | - | + | - | IgG₁ |
| 4C11 | + | - | + | - | IgG₁ |
| 1D11 | + | - | + | - | IgG_{2b} |
| 4E8 | + | - | + | - | IgG_{2b} |
| 2G5 | + | - | + | - | IgG_{2b} |
| 4E6 | + | - | + | - | IgG₁ |
| 1F4 | + | - | + | - | IgG₁ |
| 2E3 | + | - | - | + | IgG₂ₐ |
| 3D8 | + | - | - | + | IgG₂ₐ |
| 4F8 | + | - | - | + | IgG₂ₐ |
| 3D2 | + | - | - | + | IgG₂ₐ |
| 1G7 | + | - | - | + | IgG₂ₐ |
| 3D4 | + | - | - | + | IgG₂ₐ |
| 4D4 | + | - | - | - | IgG₁ |
| 5G10 | + | - | + | - | IgG₁ |
| 5E9 | + | - | + | - | IgG₁ |

| | | | | | |
|---|---|---|---|---|---|
| ^{a} Isotype specificity was determined using IsoStrip tests (Boehringer-Mannheim) for murine antibody isotype determinations which were conducted according to manufacturer's instructions. | | | | | |

In another embodiment, prostate cells are detected using a monoclonal antibody specific for a prostate cell-specific marker selected from the group consisting of prostate-specific antigen (PSA), prostate secretory protein (PSP), prostate acid phosphatase (PAP), human glandular kallekrein 2 (HK-2), prostate stem cell antigen (PSCA), and PTI-1.

In yet another embodiment, prostate cells are detected using the PR-1 monoclonal antibody (ATCC Accession No. HB-11145; Pastan, U.S. Patent No. 5,489,525).

Further, a portion of an antibody specific for a prostate cell marker, including purified fragments of the monoclonal antibodies having at least a portion of an antigen binding region, including such as Fv, F(ab')₂, Fab fragments (Harlow and Lane, 1988, Antibody, Cold Spring Harbor), single chain antibodies (U.S. Patent 4,946,778), chimeric or humanized antibodies (Morrison *et al*., 1984, Proc. Natl. Acad. Sci. USA 81:6851; Newuberger *et al*., 1984 Nature 81:6851) and complementarity determining regions (CDR) can be used in the present invention. Mimetics of the antibodies can also be used in the present invention.

The prostate cell marker-specific antibody or portion thereof can, in turn, be detected by having the antibody labeled directly or indirectly with a detectable marker. Alternatively, the prostate cell marker-specific antibody ("first antibody") can be contacted with a second antibody which is specific for the prostate cell marker-specific antibody. This second antibody can be labeled directly or indirectly with a detectable marker. Such detectable markers include, but are not limited to, a radioactive moiety, a substrate converting enzyme, fluorescent marker, biotin, and the like. For a general review, see, Antibodies, A Laboratory Manual, 1988, Harlow et al., Cold Spring Harbor Laboratory Press, Cold Spring Harbor, New York.

In an alternative embodiment, prostate cells can be detected by measuring the levels of nucleic acids and related nucleic acid sequences and subsequences, including complementary sequences, encoding a prostate cell-specific marker. For example, a cDNA encoding PSMA has been cloned. Israeli et al., 1993, Cancer Res. 53:227-230. The prostate cell-specific marker nucleic acid sequences, or subsequences thereof, comprising at least 8 nucleotides, can be used as hybridization probes. Hybridization assays can be used to detect the expression of the prostate cell-specific marker, and thus, detect prostate cells. In particular, such a hybridization assay is carried out by a method comprising contacting a sample containing nucleic acid with a nucleic acid probe capable of hybridizing to PSMA, under conditions such that hybridization can occur, and detecting or measuring any resulting hybridization.

Once the prostate cell has been identified, the prostate cell is screened for flt-4 expression. Flt-4 expression can be detected by any method known to those of skill in the art. For example, flt-4 expression can be detected using an antibody specific for flt-4 or using a fragment comprising the binding domain of an antibody specific for flt-4. Such antibodies are well known in the art and can be produced using methods well known in the art without undue experimentation, see Section 5.2.3, *infra*. In a preferred embodiment, flt-4 expression is detected using the anti-flt 4 antibody obtained from Santa Cruz Corp. (Santa Cruz, CA). Further, a portion of an antibody specific for a prostate cell marker, including purified fragments of the monoclonal antibodies having at least a portion of an antigen binding region, including such as Fv, F(ab')₂, Fab fragments (Harlow and Lane, 1988, Antibody, Cold Spring Harbor), single chain antibodies (U.S. Patent 4,946,778), chimeric or humanized antibodies (Morrison *et al*., 1984, Proc. Natl. Acad. Sci. USA 81:6851; Newuberger *et al*., 1984 Nature 81:6851) and complementarity determining regions (CDR) can be used in the present invention. Mimetics of the antibodies can also be used in the present invention.

The immunoassays which can be used include but are not limited to competitive and non-competitive assay systems using techniques such as Western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few known in the art.

The flt-4 antibody or portion thereof can, in turn, be detected by having the antibody labeled directly or indirectly with a detectable marker. Alternatively, the flt-4 antibody ("first antibody") can be contacted with a second antibody which is specific for the flt-4 antibody. This second antibody can be labeled directly or indirectly with a detectable marker. Such detectable markers include, but are not limited to, a radioactive moiety, a substrate converting enzyme, fluorescent marker, biotin, and the like. For a general review, see, Antibodies, A Laboratory Manual, 1988, Harlow et al., Cold Spring Harbor Laboratory Press, Cold Spring Harbor, New York.

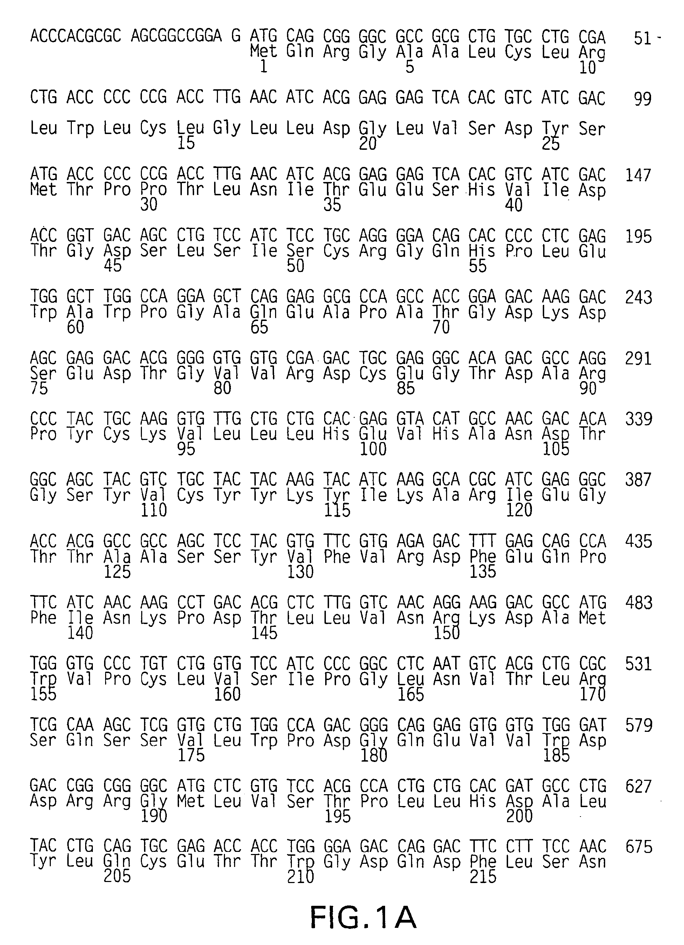

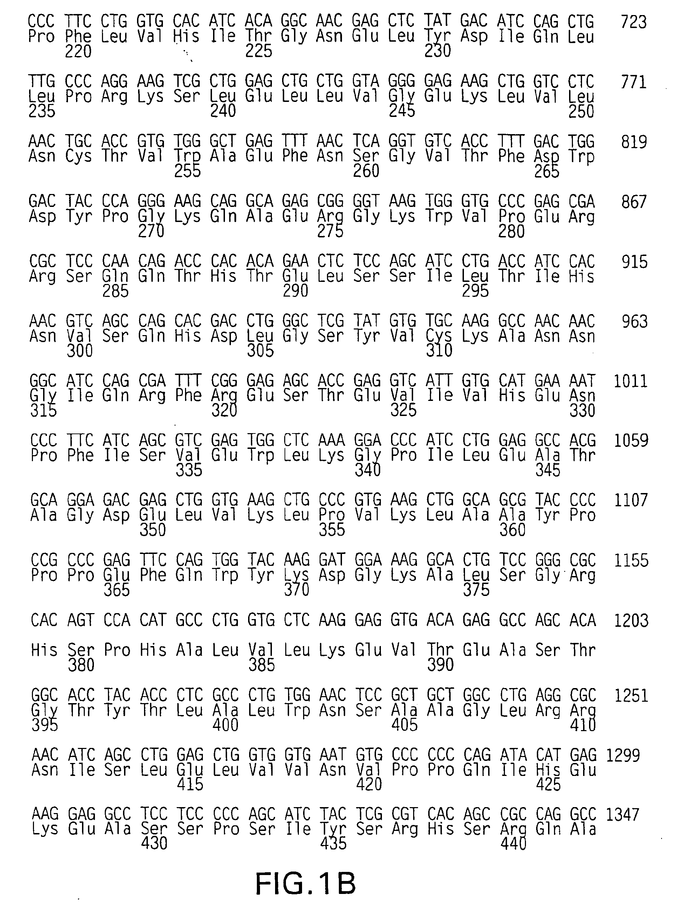

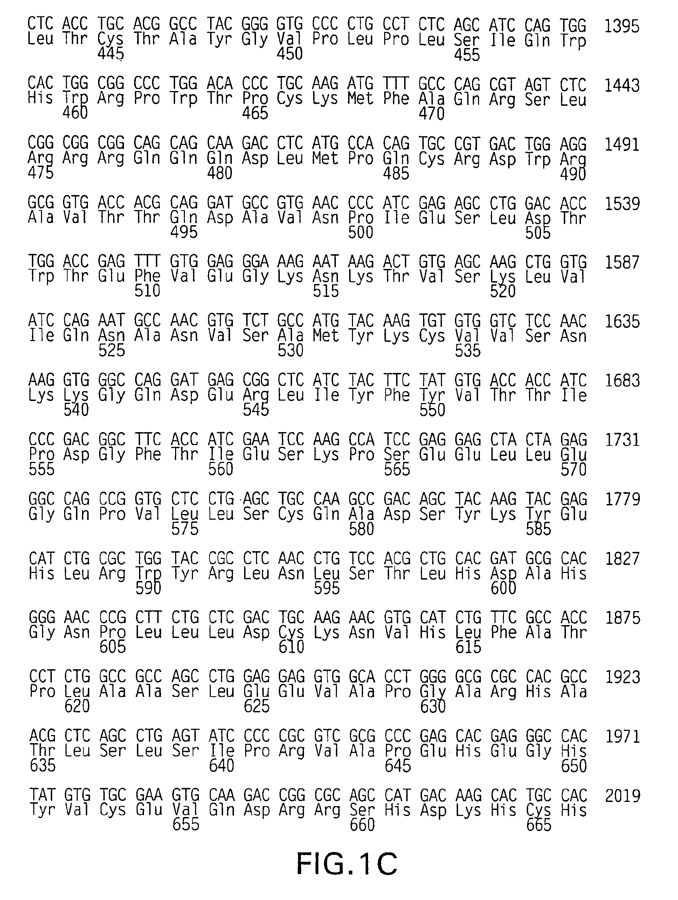

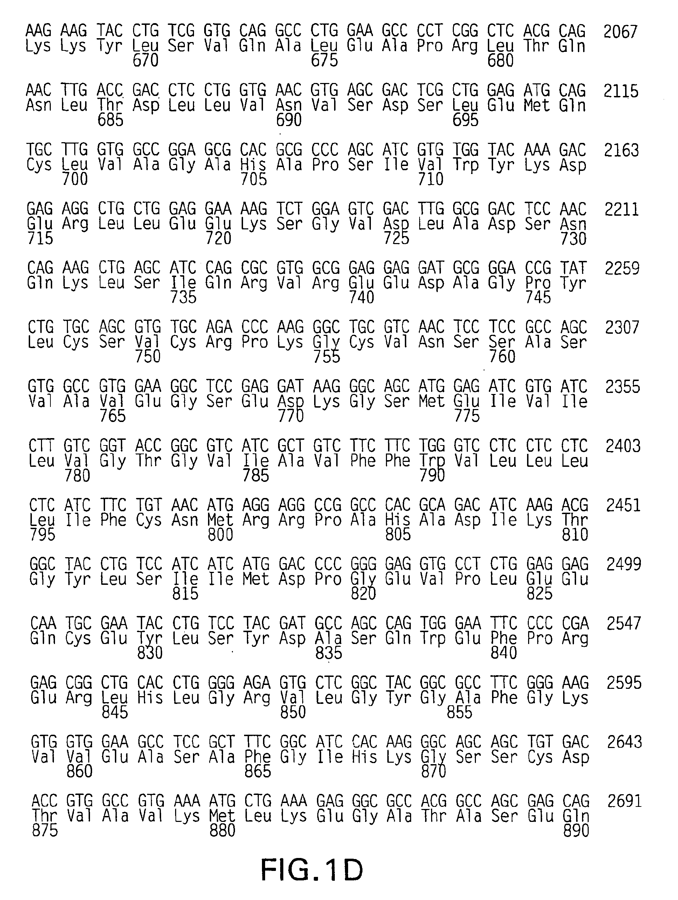

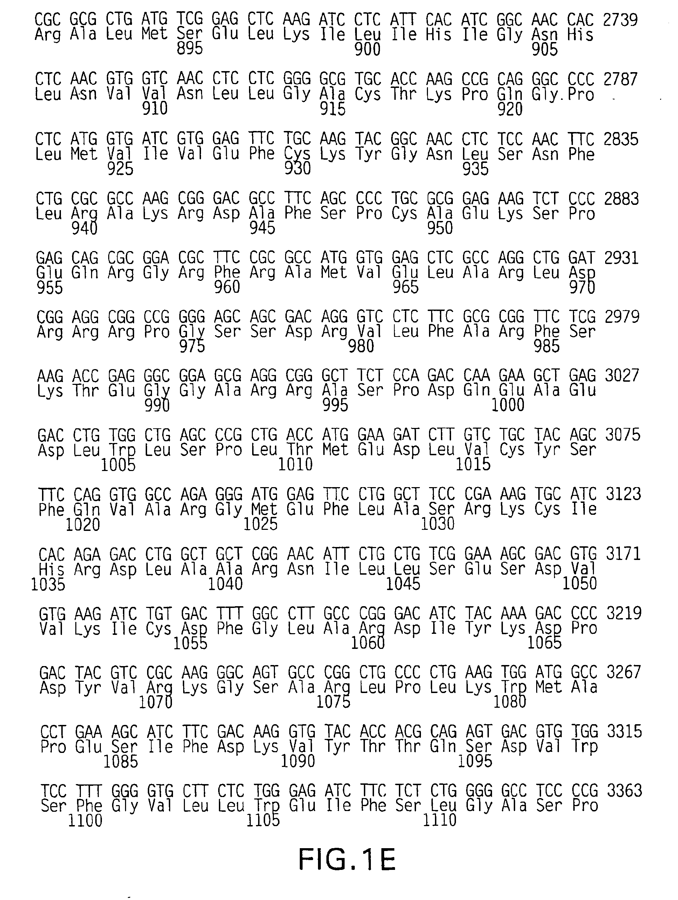

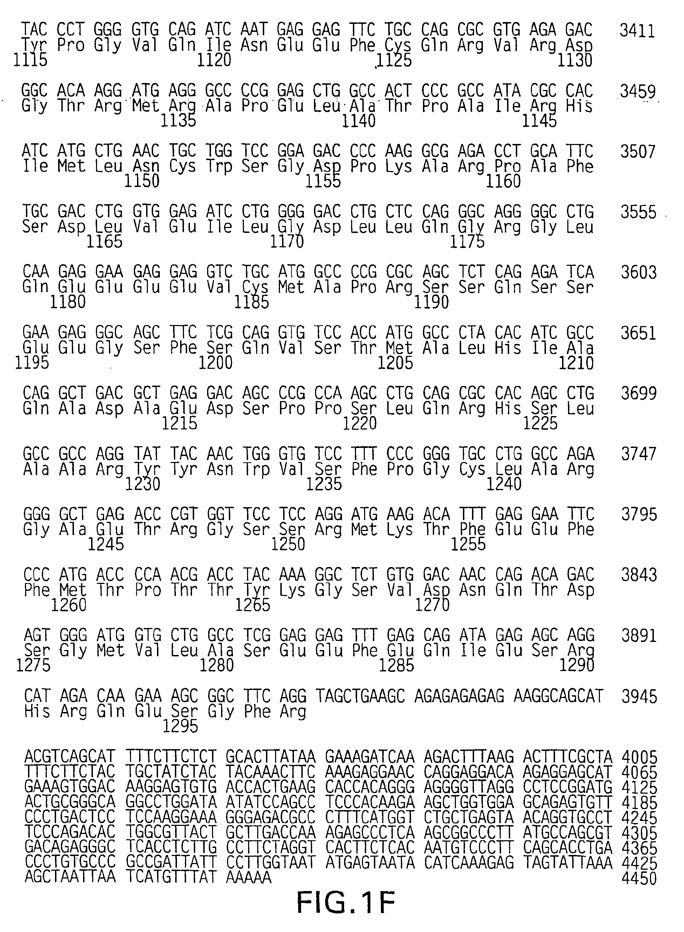

Flt-4 expression can also be detected by measuring the levels of nucleic acids and related nucleic acid sequences and subsequences, including complementary sequences, encoding flt-4. A cDNA encoding flt-4 has been cloned (Galland et al., 1992, Genomics 13(2):475-478) and the nucleotide and amino acid sequences of flt-4 are set forth in Figs. 1A-F (SEQ ID NOS:1 and 2). The flt-4 nucleic acid sequences, or subsequences thereof, comprising at least 8 nucleotides, can be used as hybridization probes. Hybridization assays can be used to detect the expression of flt-4 in a prostate cell, and thus, detect prostate cells with metastatic potential. In particular, such a hybridization assay is carried out by a method comprising contacting a sample containing nucleic acid with a nucleic acid probe capable of hybridizing to flt-4, under conditions such that hybridization can occur, and detecting or measuring any resulting hybridization.

In a preferred embodiment of the invention, the flt-4 expressing prostate cells can be detected using a flow cytometer. Fluorescence activated cell sorting (FACS) flow cytometry is a common technique for antibody based cell detection and separation. Typically, detection and separation by flow cytometry is performed as follows. A sample containing the cells of interest is contacted with fluorochrome-conjugated antibodies, which allows for the binding of the antibodies to the specific cell marker, such as PSMA and flt-4. The bound cells are then washed by one or more centrifugation and resuspension steps. The cells are then run through a FACS which separates the cells based on the different fluorescence characteristics imparted by the cell-bound fluorochrome. FACS systems are available in varying levels of performance and ability, including multicolor analysis which is preferred in the present invention. For a general review of flow cytometry, see Parks et al., 1986, Chapter 29:Flow Cytometry and fluorescence activated cell sorting (FACS) in: Handbook of Experimental Immunology, Volume 1:Immunochemistry, Weir et al. (eds.), Blackwell Scientific Publications, Boston, MA. Prostate cells are typically obtained from a body fluid sample, including blood, semen and urine.

In one aspect of this embodiment of the present invention, cells in a body fluid sample are first contacted with an antibody specific to a prostate cell-specific marker and with an antibody specific for flt-4. These "first" antibodies can be labeled either directly or indirectly with, *e.g.*, a fluorescent marker or a biotin. Alternatively, these "first" antibodies can be reacted with a "second" antibody which is specific for the first antibody, and which second antibody is labeled either directly or indirectly with, *e.g.*, a fluorescent marker. In another aspect, the "first" antibody, which is specific for PSMA, is directly labeled with biotin and the "second" antibody, which is specific for biotin, is directly labeled with a fluorescent marker; the "first" antibody, which is specific for flt-4, is indirectly labeled with a fluorescent marker. The antibody-contacted cells are then assayed in a flow cytometer.

The fluorescent label associated with the prostate cells and the fluorescent label associated with the flt-4 need to fluoresce at different wavelengths, such that the prostate cells and the prostate cells expressing flt-4 can be distinguished. The fluorescence detected by the flow cytometer at the respective different wavelengths allows for the detection of flt-4-expressing prostate cells in the sample by the characteristic profile of forward and side scatter of the cells based on their fluorescence.

In one aspect of this embodiment of the invention, when the body fluid is semen, the cells from such semen sample can be stained with a fluorescent dye specific for DNA, such that the haploid sperm cells can be distinguished from the diploid prostate cells. The haploid cells can then be removed from analysis by specific gating, *i.e.*, DNA signal histogram. However, DNA staining of the cell sample is not always necessary and may depend on the sample in terms of the number of cells and the quality of the sample, such as the number of cells present.

Other detection and separation techniques besides flow cytometry can also provide for the detection of flt-4-expressing prostate cells in a fast manner. One such method is biotin-avidin based separation by affinity chromatography. Typically, such a technique is performed by incubating the sample of cells with biotin-conjugated antibodies to specific markers, such as PSMA and flt-4, followed by passage through an avidin column. Biotin-antibody-cell complexes bind to the column via the biotin-avidin interaction, while other cells pass through the column. The specificity of the biotin-avidin system is well suited for rapid positive detection and separation.

In another preferred embodiment of the present invention, flt-4 expressing prostate cells can be detected using a laser scanning cytometer. Typically, laser scanning cytometry is performed as follows. A sample containing the cells of interest, *e.g.*, prostate gland biopsy or lymph node biopsy, is fixed on a slide and the cells are contacted with fluorochrome-conjugated antibodies, which allows for the binding of the antibodies to the prostate cell-specific marker and flt-4. The bound cells are then washed and the slide examined using a laser scanning cytometer, which allows for the identification of prostate cells, flt-4 expressing cells and prostate cells expressing flt-4 based on the different fluorescence characteristics imparted by the cell-bound fluorochrome.

Yet another method is magnetic separation using antibody-coated magnetic beads. Kemmner et al., 1992, J. Immunol. Methods 147:197-200; Racila et al., 1998, Proc. Natl. Acad. Sci. USA 95:4589-4594.

In an alternative embodiment, prostate cells expressing flt-4 can be detected by contacting prostate cells with an antibody specific for a flt-4:VEGF-C/D complex. In particular, such an immunoassay is carried out by a method comprising contacting a sample derived from a patient containing prostate cells with an anti-flt-4:VEGF-C/D complex antibody under conditions such that immunospecific binding can occur, and detecting or measuring the amount of any immunospecific binding by the antibody.

The immunoassays which can be used include but are not limited to competitive and non-competitive assay systems using techniques such as Western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few known in the art.

Kits for diagnostic use are also provided in the present invention, that comprise in one or more containers an anti-flt-4:VEGF-C/D complex antibody or an anti-flt-4 antibody, or an antibody directed to a prostate cell-specific marker, and, optionally, a labeled binding partner to the antibody. Alternatively, the antibody can be labeled with a detectable marker, *e.g.*, a chemiluminescent, enzymatic, fluorescent, or radioactive moiety. A kit is also provided that comprises in one or more containers a nucleic acid probe capable of hybridizing to flt-4 and/or a prostate cell-specific marker encoding nucleic acids, *e.g.*, PSMA mRNA. In a specific embodiment, a kit can comprise in one or more containers a pair of primers (*e*.*g*., each in the size range of 6-30 nucleotides) that are capable of priming amplification [*e*.*g*., by polymerase chain reaction (see, *e*.*g*., Innis et al., 1990, PCR Protocols, Academic Press, Inc., San Diego, CA), ligase chain reaction (EP 320,308) use of Qβ replicase, cyclic probe reaction, or other methods known in the art] under appropriate reaction conditions of at least a portion of a flt-4 and/or a prostate cell-specific marker encoding nucleic acids. A kit can optionally further comprise in a container a predetermined amount of a purified flt-4:VEGF-C/D complex, or flt-4 and/or the prostate cell-specific marker or encoding nucleic acids thereof, *e.g.*, for use as a standard or control.

### 5.2. THERAPEUTICS AND USES THEREOF

The present invention also encompasses a method for treatment, inhibition or prevention of secondary prostate tumor metastases by administration of a therapeutic molecule or composition (termed herein "Therapeutic") which molecule or composition inhibits the expression of the *flt-4* gene product or inhibits a function of wild-type flt-4, *e.g.,* flt-4 binding to VEGF-C/D (flt-4:VEGF-C/D complex formation) or tyrosine kinase activity. Such "Therapeutics" include, but are not limited to, the flt-4 protein, and analogs and derivatives (including fragments) thereof (as described *infra*); antibodies thereto (as described *infra*); nucleic acids encoding flt-4, and analogs or derivatives thereof (*e*.*g*., as described *infra*); flt-4 antisense nucleic acids, and agents that inhibit flt-4 expression or activity, *e*.*g*., flt-4 binding to VEGF-C/D and/or tyrosine kinase activity (*i*.*e*., antagonists). In a specific embodiment, the antagonist of flt-4 activity is a peptidomimetic or peptide analog or organic molecule that binds to flt-4. Such an antagonist can be identified by binding assays selected from among those known in the art.

Not intending to be limited to a particular mechanism of action, the surprising discovery that forms, in part, a basis of the present invention is that although it was known that VEGF-C and its binding receptor flt-4 are implicated in the promotion of lymphoangiogenesis, it was not known that VEGF-C and flt-4 are involved in an autocrine feedback loop resulting in the promotion and formation of secondary prostate tumor metastases. Further, the inventor discovered that the expression of flt-4 in a prostate cell indicates that such prostate cell is a cancerous prostate cell that has metastatic potential or is a secondary tumor metastasis of a primary prostate tumor, or is derived therefrom. Thus, in accordance with the present invention, secondary prostate tumors are treated or their growth inhibited or prevented by administration of a Therapeutic that inhibits flt-4 expression or flt-4 activity, *e.g.*, flt-4:VEGF-C/D complex formation. In a specific embodiment, the expression of flt-4 is inhibited by administration of an antibody specific for the extracellular domain of flt-4 or by the administration of a nucleic acid comprising a nucleotide sequence complementary to a nucleotide sequence encoding a flt-4 protein. In another specific embodiment, flt-4 function is inhibited by administration of a flt-4 protein or fragment comprising the VEGF-C/D binding region, or a nucleic acid encoding said protein or fragment, which protein or fragment acts as a competitive inhibitor of VEGF-C/D-flt-4 binding.

Therapeutics that antagonize (*i*.*e*., reduce or inhibit) flt-4 expression or flt-4 activity include, but are not limited to, flt-4 or an analog, derivative or fragment of flt-4; anti-flt-4:VEGF-C/D complex antibodies (*e.g.*, antibodies specific for a flt-4:VEGF-C/D complex, or a fragment or derivative of the antibody containing the binding region thereof; anti-flt-4 antibodies, or a fragment or derivative of such antibody containing the binding region thereof; nucleic acids encoding flt-4; flt-4 antisense nucleic acids; and flt-4 nucleic acids that are dysfunctional due to, *e.g.,* a heterologous (non-flt-4) insertion within the flt-4 coding sequence, that are used to "knockout" endogenous flt-4 function by homologous recombination, see, *e.g.*, Capecchi, 1989, Science 244:1288-1292.

In another embodiment, a Therapeutic of the present invention is a cancer vaccine. Such vaccines include, but are not limited to, dendritic cells or activated T cells. Dendritic cells can be obtained from human donors and once exposed to flt-4 antigen or antigenic fragment, are administered to a prostate cancer patient to activate relevant T cell responses *in vivo.* Alternatively, the dendritic cells are exposed to flt-4 antigen or antigenic fragment *in vitro* and incubated with primed or unprimed T cells to activate the relevant T cell responses *in vitro.* The activated T cells are then administered to a prostate cancer patient. In either alternative, dendritic cells are used to elicit an immunotherapeutic growth inhibiting response against a metastatic prostate tumor.

In a specific embodiment of the present invention, a nucleic acid containing a portion of a flt-4 gene in which flt-4 sequences flank (are both 5' and 3' to) a different gene sequence, is used as a flt-4 antagonist, or to promote flt-4 inactivation by homologous recombination (see also, Koller and Smithies, 1989, Proc. Natl. Acad. Sci. USA 86:8932-8935; Zijlstra et al., 1989, Nature 342: 435-438). Additionally, mutants or derivatives of flt-4 that have greater affinity for VEGF-C/D than wild type flt-4 may be administered to compete with wild type flt-4 protein for VEGF-C/D binding, thereby reducing the levels of VEGF-C/D complexes with wild type flt-4, which results in the inhibition of the autocrine feedback loop. Other Therapeutics that inhibit flt-4 activity, *e*.*g*., flt-4:VEGF-C/D complex function can be identified by use of known convenient *in vitro* assays, *e*.*g*., based on their ability to inhibit flt-4:VEGF-C/D binding (complex formation), or as described in Section 5.3, *infra*.

In specific embodiments, Therapeutics that antagonize flt-4 expression or activity are administered therapeutically, including prophylactically, in the treatment or inhibition of prostate cancer. A more specific embodiment of the present invention is directed to a method of reducing flt-4 expression or flt-4 activity by targeting mRNAs that express the flt-4 protein. RNA therapeutics currently fall within three classes, antisense species, ribozymes, or RNA aptamers (Good et al., 1997, Gene Therapy 4:45-54).

Antisense oligonucleotides have been the most widely used. By way of example, but not limitation, antisense oligonucleotide methodology to reduce flt-4 expression is presented below in Subsection 5.2.2, *infra*. Ribozyme therapy involves the administration, induced expression, etc. of small RNA molecules with enzymatic ability to cleave, bind, or otherwise inactivate specific RNAs, to reduce or eliminate expression of particular proteins (Grassi and Marini, 1996, Annals of Medicine 28:499-510; Gibson, 1996, Cancer and Metastasis Reviews 15:287-299). At present, the design of "hairpin" and "hammerhead" RNA ribozymes is necessary to specifically target a particular mRNA such as that for flt-4. RNA aptamers are specific RNA ligand proteins, such as for Tat and Rev RNA (Good et al., 1997, Gene Therapy 4:45-54) that can specifically inhibit their translation. Aptamers specific for flt-4 can be identified by many methods well known in the art, for example, by affecting the formation of a complex in the protein-protein interaction assay described in Section 5.4.1, *infra*.

In another embodiment, the activity or levels of flt-4 are reduced by administration of an antibody that immunospecifically binds to flt-4, or a fragment or a derivative of the antibody containing the binding domain thereof. Such antibodies are described in Section 5.2.3, *infra*. In a preferred embodiment, the anti-flt-4 antibody is obtained from Santa Cruz Corp. (Santa Cruz, CA).

Generally, administration of products of species origin or species reactivity (in the case of antibodies) that is the same species as that of the patient is preferred. Thus, in a preferred embodiment, a human flt-4 protein, or derivative, homolog or analog thereof; nucleic acids encoding human flt-4 or a derivative, homolog or analog thereof; an antibody to a human flt-4 or to a human flt-4:VEGF-C/D complex, or a derivative thereof; or other human agents that affect flt-4 expression or activity, are therapeutically or prophylactically administered to a human patient.

Preferably, suitable *in vitro* or *in vivo* assays are utilized to determine the effect of a specific Therapeutic and whether its administration is indicated for treatment of the affected tissue or individual.

In various specific embodiments, *in vitro* assays can be carried out with representative cells of cell types involved in a patient's disorder, to determine if a Therapeutic has a desired effect upon such cell types.

Therapeutics for use in therapy can be tested in suitable animal model systems prior to testing in humans, including, but not limited to, rats, mice, chicken, cows, monkeys, rabbits, etc. For *in vivo* testing, prior to administration to humans, any animal model system known in the art may be used, including those described in Section 5.6. Illustrative non-limiting examples of models of prostate cancer include those rodent models, human xenograft models, transgenic and reconstitution models, and those spontaneous prostate carcinoma models in dogs and primates listed in Waters et al., 1998, The Prostate 36:47-48; Lucia et al., 1998, The Prostate 36:49-55; Stearns et al., 1998, The Prostate 36:56-58; Green et al., 1998, The Prostate 36:59-63; and Waters et al., 1998, The Prostate 36:64-67, respectively.

### 5.2.1. GENE THERAPY

In a specific embodiment of the present invention, nucleic acids comprising a sequence encoding flt-4, or a functional derivative thereof, are administered to inhibit flt-4 activity, *e.g.*, flt-4:VEGF-C/D complex activity or formation by way of gene therapy. In more specific embodiments, a nucleic acid encoding the extracellular domain of flt-4 is administered by way of gene therapy. Gene therapy refers to therapy performed by the administration of a nucleic acid to a subject. In this embodiment of the present invention, the nucleic acid expresses its encoded protein(s) that mediates a therapeutic effect by modulating flt-4 activity by interfering with flt-4:VEGF-C/D complex activity or formation. Any of the methods for gene therapy available in the art can be used according to the present invention. Exemplary methods are described below.

For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, 1993, Science 260:926-932; Morgan and Anderson, 1993, Ann. Rev. Biochem. 62:191-217; and May, 1993, TIBTECH 11:155-215. Methods commonly known in the art of recombinant DNA technology which can be used are described in Ausubel et al., eds., 1993, Current Protocols in Molecular Biology, John Wiley & Sons, NY; and Kriegler, 1990, Gene Transfer and Expression, A Laboratory Manual, Stockton Press, NY.

In a preferred aspect, the Therapeutic comprises a flt-4 nucleic acid that is part of an expression vector that expresses a fragment of a flt-4 protein lacking at least a part of the intracellular domain in a suitable host such that the flt-4 fragment no longer has tyrosine kinase activity but still is able to bind to VEGF-C or VEGF-D. Such a fragment of flt-4 is unable to transduce the intracellular signal which normally occurs upon ligand binding, and thus, binding of VEGF-C or VEGF-D to this fragment of flt-4 will not result in the promotion of angiogenesis and autocrine proliferation of prostate cancer. In particular, such a nucleic acid has a promoter operably linked to the flt-4 coding region, said promoter being inducible or constitutive, and optionally, tissue-specific. In another particular embodiment, a nucleic acid molecule is used in which the flt-4 coding sequence, and any other desired sequences, are flanked by regions that promote homologous recombination at a desired site in the genome, thus providing for intra-chromosomal expression of the flt-4 fragment encoding nucleic acid (Koller and Smithies, 1989, Proc. Natl. Acad. Sci. USA 86:8932-8935; Zijlstra et al., 1989, Nature 342:435-438). In another preferred aspect, the nucleic acid is an antisense nucleic acid that inhibits the expression of flt-4.

Delivery of the nucleic acid into a patient may be either direct, in which case the patient is directly exposed to the nucleic acid or nucleic acid-carrying vector, or indirect, in which case, cells are first transformed with the nucleic acid *in vitro,* then transplanted into the patient. These two approaches are known, respectively, as *in vivo* or *ex vivo* gene therapy.

In a specific embodiment, the nucleic acid is directly administered *in vivo,* where it is expressed to produce the encoded product. This can be accomplished by any of numerous methods known in the art, *e.g.*, by constructing it as part of an appropriate nucleic acid expression vector and administering it so that it becomes intracellular, *e.g.*, by infection using a defective or attenuated retroviral or other viral vector (U.S. Patent No. 4,980,286), or by direct injection of naked DNA, or by use of microparticle bombardment (*e.g.*, a gene gun; Biolistic, Dupont), or coating with lipids or cell-surface receptors, or through use of transfecting agents, by encapsulation in liposomes, microparticles, or microcapsules, or by administering it in linkage to a peptide that is known to enter the nucleus, or by administering it in linkage to a ligand subject to receptor-mediated endocytosis that can be used to target cell types specifically expressing the receptors (*e.g.*, Wu and Wu, 1987, J. Biol. Chem. 262:4429-4432), etc. In another embodiment, a nucleic acid-ligand complex can be formed in which the ligand comprises a fusogenic viral peptide that disrupts endosomes, allowing the nucleic acid to avoid lysosomal degradation. In yet another embodiment, the nucleic acid can be targeted *in vivo* for cell specific uptake and expression, by targeting a specific receptor (see, *e.g.*, International Patent Publications WO 92/06180; WO 92/22635; WO 92/20316; WO 93/14188; and WO 93/20221. Alternatively, the nucleic acid can be introduced intracellularly and incorporated within host cell DNA for expression, by homologous recombination (Koller and Smithies, 1989, Proc. Natl. Acad. Sci. USA 86:8932-8935; Zijlstra et al., 1989, Nature 342:435-438).

In a specific embodiment, a viral vector that contains the flt-4 encoding nucleic acid is used. In another specific embodiment, a viral vector that contains a flt-4 antisense nucleic acid is used. For example, a retroviral vector can be used (Miller et al., 1993, Meth. Enzymol. 217:581-599). These retroviral vectors have been modified to delete retroviral sequences that are not necessary for packaging of the viral genome and integration into host cell DNA. The flt-4 encoding or antisense nucleic acids to be used in gene therapy is/are cloned into the vector, which facilitates delivery of the gene into a patient. More detail about retroviral vectors can be found in Boesen et al., 1994, Biotherapy 6:291-302, which describes the use of a retroviral vector to deliver the mdr1 gene to hematopoetic stem cells in order to make the stem cells more resistant to chemotherapy. Other references illustrating the use of retroviral vectors in gene therapy are Clowes et al., 1994, J. Clin. Invest. 93:644-651; Kiem et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are the liver, the central nervous system, endothelial cells (such as prostate cells) and muscle. Adenoviruses have the advantage of being capable of infecting non-dividing cells. Kozarsky and Wilson, 1993, Current Opinion in Genetics and Development 3:499-503, discuss adenovirus-based gene therapy. The use of adenovirus vectors to transfer genes to the respiratory epithelia of rhesus monkeys has been demonstrated by Bout et al., 1994, Human Gene Therapy 5:3-10. Other instances of the use of adenoviruses in gene therapy can be found in Rosenfeld et al., 1991, Science 252:431-434; Rosenfeld et al., 1992, Cell 68:143-155; and Mastrangeli et al., 1993, J. Clin. Invest. 91:225-234.

Adeno-associated virus (AAV) has also been proposed for use in gene therapy (Walsh et al., 1993, Proc. Soc. Exp. Biol. Med. 204:289-300.

Another approach to gene therapy involves transferring a gene into cells in tissue culture by methods such as electroporation, lipofection, calcium phosphate-mediated transfection, or viral infection. Usually, the method of transfer includes the transfer of a selectable marker to the cells. The cells are then placed under selection to isolate those cells that have taken up and are expressing the transferred gene from these that have not. Those cells are then delivered to a patient.

In this embodiment, the nucleic acid is introduced into a cell prior to administration *in vivo* of the resulting recombinant cell. Such introduction can be carried out by any method known in the art including, but not limited to, transfection by electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, *e.g.*, Loeffler and Behr, 1993, Meth. Enzymol. 217:599-618; Cohen et al., 1993, Meth. Enzymol. 217:618-644; Cline, 1985, Pharmac. Ther. 29:69-92) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably, is heritable and expressible by its cell progeny.

The resulting recombinant cells can be delivered to a patient by various methods known in the art. In a preferred embodiment, epithelial cells are injected, *e.g.*, subcutaneously. Recombinant blood cells (*e*.*g*., hematopoetic stem or progenitor cells) are preferably administered intravenously. The amount of cells envisioned for use depends on the desired effect, patient state, etc., and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes, blood cells such as T lymphocytes, B lymphocytes, monocytes, macrophages, neutrophils, eosinophils, megakaryocytes, and granulocytes, various stem or progenitor cells, in particular hematopoetic stem or progenitor cells, *e*.*g*., as obtained from bone marrow, umbilical cord blood, peripheral blood, fetal liver, etc.

In a preferred embodiment, the cell used for gene therapy is autologous to the patient.

In one embodiment in which recombinant cells are used in gene therapy, a flt-4 fragment encoding nucleic acid is introduced into the cells such that the gene is expressible by the cells or their progeny, and the recombinant cells are then administered *in vivo* for therapeutic effect. In a specific embodiment, stem or progenitor cells are used. Any stem and/or progenitor cells which can be isolated and maintained *in vitro* can potentially be used in accordance with this embodiment of the present invention. Such stem cells include but are not limited to hematopoetic stem cells (HSCs), stem cells of epithelial tissues such as the skin and the lining of the gut, embryonic heart muscle cells, liver stem cells (International Patent Publication WO 94/08598), and neural stem cells (Stemple and Anderson, 1992, Cell 71:973-985).

Epithelial stem cells (ESCs), or keratinocytes, can be obtained from tissues such as the skin and the lining of the gut by known procedures (Rheinwald, 1980, Meth. Cell Biol. 2A:229). In stratified epithelial tissue such as the skin, renewal occurs by mitosis of stem cells within the germinal layer, the layer closest to the basal lamina. Similarly, stem cells within the lining of the gut provide for a rapid renewal rate of this tissue. ESCs or keratinocytes obtained from the skin or lining of the gut of a patient or donor can be grown in tissue culture (Rheinwald, 1980, Meth. Cell Bio. 2A:229; Pittelkow and Scott, 1986, Mayo Clinic Proc. 61:771). If the ESCs are provided by a donor, a method for suppression of host versus graft reactivity (*e.g.*, irradiation, or drug or antibody administration to promote moderate immunosuppression) can also be used.

With respect to hematopoetic stem cells (HSCs), any technique that provides for the isolation, propagation, and maintenance *in vitro* of HSCs can be used in this embodiment of the invention. Techniques by which this may be accomplished include (a) the isolation and establishment of HSC cultures from bone marrow cells isolated from the future host, or a donor, or (b) the use of previously established long-term HSC cultures, which may be allogeneic or xenogeneic. Non-autologous HSCs are used preferably in conjunction with a method of suppressing transplantation immune reactions between the future host and patient. In a particular embodiment of the present invention, human bone marrow cells can be obtained from the posterior iliac crest by needle aspiration (see, *e.g.*, Kodo et al., 1984, J. Clin. Invest. 73: 1377-1384). In a preferred embodiment of the present invention, the HSCs can be made highly enriched or in substantially pure form. This enrichment can be accomplished before, during, or after long-term culturing, and can be done by any technique known in the art. Long-term cultures of bone marrow cells can be established and maintained by using, for example, modified Dexter cell culture techniques (Dexter et al., 1977, J. Cell Physiol. 91:335) or Witlock-Witte culture techniques (Witlock and Witte, 1982, Proc. Natl. Acad. Sci. USA 79:3608-3612).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

Additional methods can be adapted for use to deliver a nucleic acid encoding a fragment of flt-4 that binds to VEGF-C/D but lacks tyrosine kinase activity.

### 5.2.2. USE OF ANTISENSE OLIGONUCLEOTIDES FOR SUPPRESSION OF FLT-4 EXPRESSION

In a specific embodiment of the present invention, flt-4 expression or activity is inhibited by use of flt-4 antisense nucleic acids. The present invention provides the therapeutic or prophylactic use of nucleic acids of at least six nucleotides that are antisense to a gene or cDNA encoding flt-4, or a portion thereof. A flt "antisense" nucleic acid as used herein refers to a nucleic acid capable of hybridizing to a portion of a flt-4 RNA (preferably mRNA) by virtue of some sequence complementarity. The antisense nucleic acid may be complementary to a coding and/or noncoding region of a flt-4 mRNA. One illustrative but non-limiting example of a flt-4 antisense nucleic acid comprises the nucleotide sequence 5'-GGCGCCCCGCTGCAT-3' (SEQ ID NO:3). Such antisense nucleic acids that inhibit expression of flt-4 have utility as Therapeutics, and can be used in the treatment, inhibition or prevention of prostate cancer as described *supra*.

The antisense nucleic acids of the invention can be oligonucleotides that are double-stranded or single-stranded, RNA or DNA, or a modification or derivative thereof, which can be directly administered to a cell, or which can be produced intracellularly by transcription of exogenous, introduced sequences.

In another embodiment, the present invention is directed to a method for inhibiting the expression of flt-4 nucleic acid sequences, in a prokaryotic or eukaryotic cell, comprising providing the cell with an effective amount of a composition comprising a flt-4 antisense nucleic acid, or a derivative thereof, of the invention.

The flt-4 antisense nucleic acids are of at least six nucleotides and are preferably oligonucleotides, ranging from 6 to about 200 nucleotides. In specific aspects, the oligonucleotide is at least 10 nucleotides, at least 15 nucleotides, at least 100 nucleotides, or at least 200 nucleotides. The oligonucleotides can be DNA or RNA or chimeric mixtures, or derivatives or modified versions thereof, and either single-stranded or double-stranded. The oligonucleotide can be modified at the base moiety, sugar moiety, or phosphate backbone. The oligonucleotide may include other appending groups such as peptides, agents facilitating transport across the cell membrane (see, *e.g.*, Letsinger et al., 1989, Proc. Natl. Acad. Sci. U.S.A. 86:6553-6556; Lemaitre et al., 1987, Proc. Natl. Acad. Sci. 84:648-652; International Patent Publication No. WO 88/09810) or blood-brain barrier (see, *e.g.*, International Patent Publication No. WO 89/10134), hybridization-triggered cleavage agents (see, *e.g.*, Krol et al., 1988, BioTechniques 6:958-976), or intercalating agents (see, *e.g.*, Zon, 1988, Pharm. Res. 5:539-549).

In a preferred aspect of the invention, a flt-4 antisense oligonucleotide is provided, preferably as single-stranded DNA. The oligonucleotide may be modified at any position in its structure with constituents generally known in the art.

The flt-4 antisense oligonucleotides may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl)uracil, 5-carboxymethylaminomethyl-2-thio-uridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5N-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

In another embodiment, the oligonucleotide comprises at least one modified sugar moiety selected from the group including, but not limited to, arabinose, 2-fluoroarabinose, xylulose, and hexose.

In yet another embodiment, the oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal, or an analog of the foregoing.

In yet another embodiment, the oligonucleotide is a 2-a-anomeric oligonucleotide. An a-anomeric oligonucleotide forms specific double-stranded hybrids with complementary RNA in which, contrary to the usual β-units, the strands run parallel to each other (Gautier et al., 1987, Nucl. Acids Res. 15:6625-6641).

The oligonucleotide may be conjugated to another molecule, *e.g.*, a peptide, hybridization-triggered cross-linking agent, transport agent, hybridization-triggered cleavage agent, etc.

Oligonucleotides of the invention may be synthesized by standard methods known in the art, *e*.*g*., by use of an automated DNA synthesizer (such as are commercially avail-able from Biosearch, Applied Biosystems, etc.). As examples, phosphorothioate oligo-nucleotides may be synthesized by the method of Stein et al. (1988, Nucl. Acids Res. 16:3209), methylphosphonate oligonucleotides can be prepared by use of controlled pore glass polymer supports (Sarin et al., 1988, Proc. Natl. Acad. Sci. U.S.A. 85:7448-7451), etc.

In a specific embodiment, the flt-4 antisense oligonucleotides comprise catalytic RNAs, or ribozymes (see, *e.g.*, International Patent Publication No. WO 90/11364; Sarver et al., 1990, Science 247:1222-1225). In another embodiment, the oligonucleotide is a 2'-0-methylribonucleotide (Inoue et al., 1987, Nucl. Acids Res. 15:6131-6148), or a chimeric RNA-DNA analog (Inoue et al., 1987, FEBS Lett. 215:327-330).

In an alternative embodiment, the flt-4 antisense nucleic acids of the invention are produced intracellularly by transcription from an exogenous sequence. For example, a vector can be introduced in vivo such that it is taken up by a cell, within which cell the vector or a portion thereof is transcribed, producing an antisense nucleic acid (RNA) of the invention. Such a vector would contain a sequence encoding a flt-4 antisense nucleic acid. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art to be capable of replication and expression in mammalian cells. Expression of the sequences encoding the flt-4 antisense RNAs can be by any promoter known in the art to act in mammalian, preferably human, cells. Such promoters can be inducible or constitutive. Such promoters include, but are not limited to, the SV40 early promoter region (Bernoist and Chambon, 1981, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42), etc.

The antisense nucleic acids of the invention comprise a sequence complementary to at least a portion of an RNA transcript of a flt-4 gene, preferably a human flt-4 gene. However, absolute complementarity, although preferred, is not required. A sequence "complementary to at least a portion of an RNA," as referred to herein, means a sequence having sufficient complementarity to be able to hybridize with the RNA, forming a stable duplex; in the case of double-stranded flt-4 antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with a flt-4 RNA it may contain and still form a stable duplex (or triplex, as the case may be). One skilled in the art can ascertain a tolerable degree of mismatch by use of standard procedures to determine the melting point of the hybridized complex.

The flt-4 antisense nucleic acid can be used to treat (or prevent) secondary prostate tumor metastases. In a preferred embodiment, a single-stranded flt-4 DNA antisense oligonucleotide is used.

Pharmaceutical compositions of the invention (see Section 5.5, *infra*), comprising an effective amount of a flt-4 antisense nucleic acid in a pharmaceutically acceptable carrier can be administered to a patient having a secondary prostate tumor metastasis.

The amount of flt-4 antisense nucleic acid that will be effective in the treatment of a particular disorder or condition will depend on the nature of the disorder or condition, and can be determined by standard clinical techniques. Where possible, it is desirable to determine the antisense cytotoxicity in vitro, and then in useful animal model systems, prior to testing and use in humans.

In a specific embodiment, pharmaceutical compositions comprising flt-4 antisense nucleic acids are administered via liposomes, microparticles, or microcapsules. In various embodiments of the invention, it may be useful to use such compositions to achieve sustained release of the flt-4 antisense nucleic acids.

### 5.2.3. ANTIBODIES TO FLT-4

According to the present invention, flt-4 or a fragment, derivative or homolog thereof, or a flt-4:VEGF-C/D complex or a fragment, derivative or homolog thereof, may be used as an immunogen to generate antibodies which immunospecifically bind such immunogen. Such antibodies include, but are not limited to, polyclonal, monoclonal, chimeric, single chain, Fab fragments, and an Fab expression library. In a specific embodiment, antibodies to human flt-4 are produced. In another specific embodiment, antibodies to a complex of human flt-4 and human VEGF-C or VEGF-D are produced. In another embodiment, a complex formed from a fragment of flt-4 and a fragment of VEGF-C or VEGF-D, which fragments contain the protein domain that interacts with the other member of the complex, are used as an immunogen for antibody production. One illustrative but non-limiting example of an anti-flt-4 antibody is the anti-flt-4 antibody obtained from Santa Cruz Corp. (Santa Cruz, CA).

In another specific embodiment, the antibody to a flt-4 protein is a bispecific antibody (see generally, *e.g.* Fanger and Drakeman, 1995, Drug News and Perspectives 8: 133-137). Such a bispecific antibody is genetically engineered to recognize both (1) a flt-4 epitope and (2) one of a variety of "trigger" molecules, *e.g.* Fc receptors on myeloid cells, and CD3 and CD2 on T cells, that have been identified as being able to cause a cytotoxic T-cell to destroy a particular target. Such bispecific antibodies can be prepared either by chemical conjugation, hybridoma, or recombinant molecular biology techniques known to the skilled artisan.

Various procedures known in the art may be used for the production of polyclonal antibodies to a flt-4 protein, or a fragment, derivative, homolog or analog of the protein or for the production of polyclonal antibodies to a complex of flt-4 and VEGF-C/D.

For production of the antibody, various host animals can be immunized by injection with a native flt-4 protein or a flt-4:VEGF-C/D complex or a synthetic version, or a derivative of the foregoing, such as a cross-linked flt-4:VEGF-C/D complex. Such host animals include, but are not limited to, rabbits, mice, rats, etc. Various adjuvants can be used to increase the immunological response, depending on the host species, and include, but are not limited to, Freund's (complete and incomplete), mineral gels such as aluminum hydroxide, surface active substances such as lysolecithin, pluronic polyols, polyanions, peptides, oil emulsions, dinitrophenol, and potentially useful human adjuvants such as bacille Calmette-Guerin (BCG) and Corynebacterium parvum.

For preparation of monoclonal antibodies directed towards a flt-4 protein or derivative, fragment, homolog or analog thereof, or a flt-4:VEGF-C/D complex, or a derivative, fragment, homolog or analog thereof, any technique that provides for the production of antibody molecules by continuous cell lines in culture may be used. Such techniques include, but are not restricted to, the hybridoma technique originally developed by Kohler and Milstein (1975, Nature 256:495-497), the trioma technique (Gustafsson et al., 1991, Hum. Antibodies Hybridomas 2:26-32), the human B-cell hybridoma technique (Kozbor et al., 1983, Immunology Today 4:72), and the EBV hybridoma technique to produce human monoclonal antibodies (Cole et al., 1985, In: Monoclonal Antibodies and Cancer Therapy, Alan R. Liss, Inc., pp. 77-96). In an additional embodiment of the invention, monoclonal antibodies can be produced in germ-free animals utilizing recent technology described in International Patent Application PCT/US90/02545.

According to the present invention, human antibodies may be used and can be obtained by using human hybridomas (Cote et al., 1983, Proc. Natl. Acad. Sci. USA 80:2026-2030) or by transforming human B cells with EBV virus in vitro (Cole et al., 1985, In: Monoclonal Antibodies and Cancer Therapy, Alan R. Liss, Inc., pp. 77-96). In fact, according to the invention, techniques developed for the production of "chimeric antibodies" (Morrison et al., 1984, Proc. Natl. Acad. Sci. USA 81:6851-6855; Neuberger et al., 1984, Nature 312:604-608; Takeda et al., 1985, Nature 314:452-454) by splicing the genes from a mouse antibody molecule specific for the flt-4 protein together with genes from a human antibody molecule of appropriate biological activity can be used; such antibodies are within the scope of this invention.

According to the present invention, techniques described for the production of single chain antibodies (U.S. Patent 4,946,778) can be adapted to produce flt-4 or flt-4:VEGF-C/D complex-specific antibodies. An additional embodiment of the invention utilizes the techniques described for the construction of Fab expression libraries (Huse et al., 1989, Science 246:1275-1281) to allow rapid and easy identification of monoclonal Fab fragments with the desired specificity for flt-4 or for flt-4:VEGF-C/D complexes, derivatives, or analogs thereof. Non-human antibodies can be "humanized" by known methods (*e.g.*, U.S. Patent No. 5,225,539).

Antibody fragments that contain the idiotypes of flt-4 or flt-4:VEGF-C/D complex can be generated by techniques known in the art. For example, such fragments include, but are not limited to, the F(ab')2 fragment which can be produced by pepsin digestion of the antibody molecule; the Fab' fragment that can be generated by reducing the disulfide bridges of the F(ab')2 fragment; the Fab fragment that can be generated by treating the antibody molecular with papain and a reducing agent; and Fv fragments.

In the production of antibodies, screening for the desired antibody can be accomplished by techniques known in the art, *e*.*g*., ELISA (enzyme-linked immunosorbent assay). To select antibodies specific to a particular domain of the flt-4 protein, or a derivative, homolog, or analog thereof, one may assay generated hybridomas for a product that binds to the fragment of the flt-4 protein, or a derivative, homolog, or analog thereof, that contains such a domain. For selection of an antibody that specifically binds a flt-4:VEGF-C/D complex, or a derivative, homolog, or analog thereof, but which does not specifically bind to the individual proteins of the flt-4:VEGF-C/D complex, or a derivative, homolog, or analog thereof, one can select on the basis of positive binding to the flt-4:VEGF-C/D complex and a lack of binding to the individual flt-4 and VEGF-C or VEGF-D proteins.

Antibodies specific to a domain of the flt-4 protein or to a domain of the flt-4:VEGF-C/D complex, or a derivative, homolog, or analog thereof, are also provided.

The foregoing antibodies can be used in methods known in the art relating to the localization and/or quantification of flt-4 or flt-4:VEGF-C/D complexes, *e.g.*, for imaging these proteins, measuring levels thereof in appropriate physiological samples, in diagnostic methods, etc. This hold true also for a derivative, homolog, or analog thereof of a flt-4 protein or of a flt-4:VEGF-C/D complex.

In another embodiment of the invention (see *infra*), an anti-flt-4 antibody or an anti-flt-4:VEGF-C/D complex antibody or a fragment thereof containing the binding domain, is a Therapeutic.

Antibodies and antigen-binding antibody fragments may also be conjugated to a heterologous protein or peptide by chemical conjugation or recombinant DNA technology. The resultant chimeric protein possesses the antigen-binding specificity of the antibody and the function of the heterologous protein. For example, a polynucleotide encoding the antigen binding region of an antibody specific for the extracellular domain of flt-4 can be genetically fused to a coding sequence for the zeta chain of the T cell receptor. After expressing this construct in T cells, the T cells are expanded *ex vivo* and infused into a prostate cancer patient. T cells expressing this chimeric protein are specifically directed to tumors that express flt-4 as a result of the antibody binding specificity and cause tumor cell killing. Alternatively, an antibody is fused to a protein which induces migration of leukocytes or has an affinity to attract other compounds to a tumor cite. A specific protein of this type is streptavidin. The binding of a streptavidin conjugated antibody to a tumor cell can be followed by the addition of a biotinylated drug, toxin or radioisotope to cause tumor specific killing.

Kits for use with such *in vitro* tumor localization and therapy methods containing the monoclonal antibodies (or fragments thereof) conjugated to any of the above types of substances can be prepared. The components of the kits can be packaged either in aqueous medium or in lyophilized form. When the monoclonal antibodies (or fragments thereof) are used in the kits in the form of conjugates in which a label or a therapeutic moiety is attached, such as a radioactive metal ion or a therapeutic drug moiety, the components of such conjugates can be supplied either in fully conjugated form, in the form of intermediates or as separate moieties to be conjugated by the user of the kit.

### 5.3. ASSAYS OF FLT-4:VEGF-C/D COMPLEXES AND DERIVATIVES AND ANALOGS THEREOF

The functional activity of flt-4, as measured by its ability to form a flt-4:VEGF-C/D complex, or a derivative, fragment or analog thereof, can be assayed by various methods. Potential antagonists of flt-4:VEGF-C/D complex formation, *e.g.*, anti-flt-4:VEGF-C/D complex antibodies and flt-4 antisense nucleic acids, can be assayed for the ability to inhibit flt-4:VEGF-C/D complex formation.

In one embodiment of the present invention, where one is assaying for the ability to bind or compete with a wild-type flt-4:VEGF-C/D complex for binding to an anti-flt-4:VEGF-C/D complex antibody, various immunoassays known in the art can be used, including but not limited to competitive and non-competitive assay systems using techniques such as radioimmunoassay, ELISA (enzyme linked immunosorbent assay), "sandwich" immunoassays, immunoradiometric assays, gel diffusion precipitin reactions, immunodiffusion assays, in situ immunoassays (using colloidal gold, enzyme or radioisotope labels), western blot analysis, precipitation reactions, agglutination assays (*e*.*g*., gel agglutination assays, hemagglutination assays), complement fixation assays, immunofluorescence assays, protein A assays, immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention.

The expression of the flt-4 gene (both endogenous and those expressed from cloned DNA containing the genes) can be detected using techniques known in the art, including but not limited to Southern hybridization (Southern, 1975, J. Mol. Biol. 98:503-517), northern hybridization (see, *e.g.*, Freeman et al., 1983, Proc. Natl. Acad. Sci. USA 80:4094-4098), restriction endonuclease mapping (Sambrook et al., 1989, Molecular Cloning, A Laboratory Manual, 2^{nd} Ed. Cold Spring Harbor Laboratory Press, New York), RNase protection assays (Current Protocols in Molecular Biology, John Wiley and Sons, New York, 1997), DNA sequence analysis, and polymerase chain reaction amplification (PCR; U.S. Patent Nos. 4,683,202, 4,683,195, and 4,889,818; Gyllenstein et al., 1988, Proc. Natl. Acad. Sci. USA 85:7652-7657; Ochman et al., 1988, Genetics 120:621-623; Loh et al., 1989, Science 243:217-220) followed by Southern hybridization with probes specific for the flt-4 gene, in various cell types. Methods of amplification other than PCR commonly known in the art can be employed. The stringency of the hybridization conditions for northern or Southern blot analysis can be manipulated to ensure detection of nucleic acids with the desired degree of relatedness to the specific probes used. Modifications to these methods and other methods commonly known in the art can be used.

Derivatives (*e.g.*, fragments), homologs and analogs of flt-4 can be assayed for binding to VEGF-C/D by any method known in the art, for example the modified yeast matrix mating test described in Section 5.4.1, *infra*; immunoprecipitation with an antibody that binds to flt-4 complexed with other proteins, followed by size fractionation of the immunoprecipitated proteins (*e.g.*, by denaturing or nondenaturing polyacrylamide gel electrophoresis); Western blot analysis, etc.

One embodiment of the invention provides a method for screening a derivative, homolog or analog of flt-4 for biological activity comprising contacting said derivative, homolog or analog of flt-4 with VEGF-C/D; and detecting the formation of a complex between said derivative, homolog or analog of flt-4 and VEGF-C/D; wherein detecting formation of said complex indicates that said derivative, homolog or analog of flt-4 has biological (*e*.*g*., binding) activity.

### 5.4. SCREENING FOR ANTAGONISTS OF FLT-4 ACTIVITY

A functional activity of flt-4 is its ability to bind its ligand, VEGF-C or VEGF-D. Thus, flt-4:VEGF-C/D complexes, and derivatives, fragments and analogs thereof, nucleic acids encoding flt-4, VEGF-C and VEGF-D as well as derivatives, fragments and analogs of the nucleic acids, can be used to screen for compounds that bind to or modulate the function of, flt-4:VEGF-C/D complex encoding nucleic acids, complex member proteins, and derivatives of the foregoing, and thus, have potential use as agonists or antagonists of flt-4:VEGF-C/D complex activity or formation. The present invention is thus directed to assays for detecting molecules that specifically bind to, or modulate the function of, flt-4, VEGF-C and VEGF-D nucleic acids, proteins or derivatives of the nucleic acids and proteins. For example, recombinant cells expressing both flt-4, VEGF-C and VEGF-D nucleic acids can be used to recombinantly produce the complexes or proteins in these assays, to screen for molecules that bind to, or interfere with, or promote flt-4:VEGF-C/D complex formation or activity. In preferred embodiments, polypeptide analogs that have superior stabilities but retain the ability to form a flt-4:VEGF-C/D complex (*e.g.*, flt-4 and VEGF-C or VEGF-D modified to be resistant to proteolytic degradation in the binding assay buffers, or to be resistant to oxidative degradation), are used to screen for modulators of flt-4 activity or flt-4:VEGF-C/D complex activity or formation. Such resistant molecules can be generated, *e*.*g*., by substitution of amino acids at proteolytic cleavage sites, the use of chemically derivatized amino acids at proteolytic susceptible sites, and the replacement of amino acid residues subject to oxidation, *i*.*e*. methionine and cysteine.

A molecule (*e.g.*, putative binding partner or modulator of flt-4:VEGF-C/D complex activity or formation) is contacted with the flt-4:VEGF-C/D complex, or fragment thereof, under conditions conducive to binding or modulation, and then a molecule that specifically bind to or modulate flt-4:VEGF-C/D complex activity or formation is identified. Similar methods can be used to screen for molecules that bind to or modulate the function of flt-4:VEGF-C/D nucleic acids or derivatives thereof.

A particular aspect of the present invention relates to identifying molecules that inhibit or promote formation or degradation of a flt-4:VEGF-C/D complex, *e*.*g*., using the method described for screening inhibitors using the modified yeast matrix mating test described in Section 5.6.1., *infra*, and International Patent Publication WO 97/47763 entitled "Identification and Comparison of Protein-Protein Interactions that Occur in Populations and Identification of Inhibitors of These Interactions", which is incorporated by reference herein in its entirety.

In one embodiment of the invention, a molecule that modulates activity of flt-4 or a complex of flt-4 and VEGF-C/D, is identified by contacting one or more candidate molecules with flt-4 in the presence of VEGF-C or VEGF-D; and measuring the amount of complex that forms between flt-4 and VEGF-C or VEGF-D; wherein an increase or decrease in the amount of complex that forms relative to the amount that forms in the absence of the candidate molecule(s) indicates that the molecule(s) modulates the activity of flt-4 or VEGF-C or VEGF-D or said complex of flt-4 and VEGF-C or VEGF-D. In preferred embodiments, a modulator is identified by administering a candidate molecule to a transgenic non-human animal expressing both flt-4 and VEGF-C or flt-4 and VEGF-D from promoters that are not the native flt-4 or the native VEGF-C or VEGF-D promoters; more preferably where the candidate molecule is also recombinantly expressed in the transgenic non-human animal. Alternatively, the method for identifying such a modulator can be carried out *in vitro,* preferably with purified flt-4, purified VEGF-C or VEGF-D, and a purified candidate molecule.

Methods that can be used to carry out the foregoing are commonly known in the art. Agents/molecules to be screened can be provided as mixtures of a limited number of specified compounds, or as compound libraries, peptide libraries and the like. Agents/molecules to be screened may also include all forms of antisera, antisense nucleic acids, etc., that can modulate flt-4:VEGF-C/D complex activity or formation.

By way of example, diversity libraries, such as random or combinatorial peptide or non-peptide libraries, can be screened for molecules that specifically bind to a flt-4:VEGF-C/D complex. Many libraries are known in the art that can be used, *e*.*g*., chemically-synthesized libraries, recombinant, *e.g.*, phage display, libraries, and *in vitro* translation-based libraries.

Examples of chemically-synthesized libraries are described in Fodor et al., 1991, Science 251:767-773; Houghten et al., 1991, Nature 354:84-86; Lam et al., 1991, Nature 354:82-84; Medynski, 1994, BioTechnology 12:709-710; Gallop et al., 1994, J. Medicinal Chemistry 37:1233-1251; Ohlmeyer et al., 1993, Proc. Natl. Acad. Sci. USA 90:10922-10926; Erb et al., 1994, Proc. Natl. Acad. Sci. USA 91:11422-11426; Houghten et al., 1992, BioTechniques 13:412; Jayawickreme et al., 1994, Proc. Natl. Acad. Sci. USA 91:1614-1618; Salmon et al., 1993, Proc. Natl. Acad. Sci. USA 90:11708-11712; International Patent Publication No. WO 93/20242; and Brenner and Lerner, 1992, Proc. Natl. Acad. Sci. USA 89:5381-5383.

Examples of phage display libraries are described in Scott and Smith, 1990, Science 249:386-390; Devlin et al., 1990, Science, 249:404-406; Christian et al., 1992, J. Mol. Biol. 227:711-718; Lenstra, 1992, J. Immunol. Meth. 152:149-157; Kay et al., 1993, Gene 128:59-65; and International Patent Publication No. WO 94/18318.

*In vitro* translation-based libraries include but are not limited to those described in International Patent Publication No. WO 91/05058; and Mattheakis et al., 1994, Proc. Natl. Acad. Sci. USA 91:9022-9026.

By way of examples of non-peptide libraries, a benzodiazepine library (*e.g.*, Bunin et al., 1994, Proc. Natl. Acad. Sci. USA 91:4708-4712) can be adapted for use. Peptoid libraries (Simon et al., 1992, Proc. Natl. Acad. Sci. USA 89:9367-9371) can also be used. Another example of a library that can be used, in which the amide functionalities in peptides have been permethylated to generate a chemically-transformed combinatorial library, is described by Ostresh et al. (1994, Proc. Natl. Acad. Sci. USA 91:11138-11142).

Screening the libraries can be accomplished by any of a variety of commonly known methods. See, *e.g.*, the following references, which disclose screening of peptide libraries: Parmley and Smith, 1989, Adv. Exp. Med. Biol. 251:215-218; Scott and Smith, 1990, Science 249:386-390; Fowlkes et al., 1992, BioTechniques 13:422-427; Oldenburg et al., 1992, Proc. Natl. Acad. Sci. USA 89:5393-5397; Yu et al., 1994, Cell 76:933-945; Staudt et al., 1988, Science 241:577-580; Bock et al., 1992, Nature 355:564-566; Tuerk et al., 1992, Proc. Natl. Acad. Sci. USA 89:6988-6992; Ellington et al., 1992, Nature 355:850-852; U.S. Patent No. 5,096,815, U.S. Patent No. 5,223,409, and U.S. Patent No. 5,198,346, all to Ladner et al.; Rebar and Pabo, 1993, Science 263:671-673; and International Patent Publication No. WO 94/18318.

In a specific embodiment, screening can be carried out by contacting the library members with a flt-4:VEGF-C/D complex (or encoding nucleic acid or derivative) immobilized on a solid phase, and harvesting those library members that bind to the protein (or encoding nucleic acid or derivative). Examples of such screening methods, termed "panning" techniques, are described by way of example in Parmley and Smith, 1988, Gene 73:305-318; Fowlkes et al., 1992, BioTechniques 13:422-427; International Patent Publication No. WO 94/18318; and in references cited hereinabove.

In a specific embodiment, fragments and/or analogs of flt-4 or VEGF-C or VEGF-D, especially peptidomimetics, are screened for activity as competitive or non-competitive inhibitors of flt-4:VEGF-C/D complex formation, which thereby inhibit flt-4:VEGF-C/D complex activity or formation.

In a preferred embodiment, molecules that bind to flt-4:VEGF-C/D complexes can be screened for using the modified yeast matrix mating test described in Section 5.4.1, *infra*.

In one embodiment, agents that modulate (*i*.*e*., antagonize or agonize) flt-4:VEGF-C/D complex activity or formation (flt-4 activity) can be screened for using a binding inhibition assay, wherein agents are screened for their ability to modulate formation of a flt-4:VEGF-C/D complex under aqueous, or physiological, binding conditions in which flt-4:VEGF-C/D complex formation occurs in the absence of the agent to be tested. Agents that interfere with the formation of flt-4:VEGF-C/D complexes are identified as antagonists of complex formation. Agents that promote the formation of flt-4:VEGF-C/D complexes are identified as agonists of complex formation. Agents that completely block the formation of flt-4:VEGF-C/D complexes are identified as inhibitors of complex formation.

Methods for screening may involve labeling the complex proteins with radioligands (*e.g.*, ¹²⁵I or ³H), magnetic ligands (*e.g.*, paramagnetic beads covalently attached to photobiotin acetate), fluorescent ligands (*e.g.*, fluorescein or rhodamine), or enzyme ligands (e.g., luciferase or beta-galactosidase). The reactants that bind in solution can then be isolated by one of many techniques known in the art, including but not restricted to, co-immunoprecipitation of the labeled complex moiety using antisera against the unlabeled binding partner (or labeled binding partner with a distinguishable marker from that used on the second labeled complex moiety), immunoaffinity chromatography, size exclusion chromatography, and gradient density centrifugation. In a preferred embodiment, the labeled binding partner is a small fragment or peptidomimetic that is not retained by a commercially available filter. Upon binding, the labeled species is then unable to pass through the filter, providing for a simple assay of complex formation.

Methods commonly known in the art are used to label at least one of the members of the flt-4:VEGF-C/D complex. Suitable labeling methods include, but are not limited to, radiolabeling by incorporation of radiolabeled amino acids, *e.g.*, ³H-leucine or ³⁵S-methionine, radiolabeling by post-translational iodination with ¹²⁵I or ¹³¹I using the chloramine T method, Bolton-Hunter reagents, etc., or labeling with ³²P using phosphorylase and inorganic radiolabeled phosphorous, biotin labeling with photobiotin-acetate and sunlamp exposure, etc. In cases where one of the members of the flt-4:VEGF-C/D complex is immobilized, *e.g.*, as described *infra*, the free species is labeled. Where neither of the interacting species is immobilized, each can be labeled with a distinguishable marker such that isolation of both moieties can be followed to provide for more accurate quantification, and to distinguish the formation of homomeric from heteromeric complexes. Methods that utilize accessory proteins that bind to one of the modified interactants to improve the sensitivity of detection, increase the stability of the complex, etc. are provided.

Typical binding conditions are, for example, but not by way of limitation, in an aqueous salt solution of 10-250 mM NaCl, 5-50 mM Tris-HCl, pH 5-8, and 0.5% Triton X-100 or other detergent that improves specificity of interaction. Metal chelators and/or divalent cations may be added to improve binding and/or reduce proteolysis. Reaction temperatures may include 4, 10, 15, 22, 25, 35, or 42 degrees Celsius, and time of incubation is typically at least 15 seconds, but longer times are preferred to allow binding equilibrium to occur. Particular flt-4:VEGF-C/D complexes can be assayed using routine protein binding assays, as described infra, to determine optimal binding conditions for reproducible binding.

The physical parameters of complex formation can be analyzed by quantification of complex formation using assay methods specific for the label used, *e*.*g*., liquid scintillation counting for radioactivity detection, enzyme activity for enzyme-labeled moieties etc. The reaction results are then analyzed utilizing Scatchard analysis, Hill analysis, and other methods commonly known in the arts (see, *e.g.*, Proteins, Structures, and Molecular Principles, 2^{nd} Edition (1993) Creighton, Ed., W.H. Freeman and Company, New York).

In a second common approach to binding assays, one of the binding species is immobilized on a filter, in a microtiter plate well, in a test tube, to a chromatography matrix, etc., either covalently or non-covalently. Proteins can be covalently immobilized using any method well known in the art, for example, but not limited to the method of Kadonaga and Tjian, 1986, Proc. Natl. Acad. Sci. USA 83:5889-5893, *i*.*e*., linkage to a cyanogen-bromide derivatized substrate such as CNBr-Sepharose 4B (Pharmacia). Where needed, the use of spacers can reduce steric hindrance by the substrate. Non-covalent attachment of proteins to a substrate include, but are not limited to, attachment of a protein to a charged surface, binding with specific antibodies, binding to a third unrelated interacting protein, etc.

In one embodiment, immobilized flt-4 is used to assay for binding to radioactively-labeled VEGF-C or VEGF-D in the presence and absence of a compound to be tested for its ability to modulate flt-4:VEGF-C/D complex formation. The binding partners are allowed to bind under aqueous, or physiological, conditions (*e.g.*, the conditions under which the original interaction was detected). Conversely, in another embodiment, VEGF-C or VEGF-D is immobilized and contacted with a labeled flt-4 protein or derivative thereof under binding conditions.

Assays of agents (including cell extracts or a library pool) for competition for binding of one member of a flt-4:VEGF-C/D complex (or derivatives thereof) with the other member of the flt-4:VEGF-C/D complex labeled by any means (*e.g.*, those means described above) are provided to screen for competitors or enhancers of flt-4:VEGF-C/D complex formation.

In specific embodiments, blocking agents to inhibit non-specific binding of reagents to other protein components, or absorptive losses of reagents to plastics, immobilization matrices, etc., are included in the assay mixture. Blocking agents include, but are not restricted to bovine serum albumin, beta-casein, nonfat dried milk, Denhardt's reagent, Ficoll, polyvinylpyrolidine, nonionic detergents (NP40, Triton X-100, Tween 20, Tween 80, etc.), ionic detergents (e.g., SDS, LDS, etc.), polyethylene glycol, etc. Appropriate blocking agent concentrations allow flt-4:VEGF-C/D complex formation.

After binding is performed, unbound, labeled protein is removed in the supernatant, and the immobilized protein retaining any bound, labeled protein is washed extensively. The amount of bound label is then quantified using standard methods in the art to detect the label as described supra.

### 5.4.1. ASSAYS FOR PROTEIN-PROTEIN INTERACTIONS

One aspect of the present invention provides methods for assaying and screening a fragment, derivative or analog of a flt-4-interacting protein for binding to a flt-4 peptide, or a fragment, derivative, homolog or analog of flt-4. Derivatives, analogs and fragments of VEGF-C or VEGF-D that interact with flt-4 or a derivative, analog or fragment of flt-4 can be identified by means of a yeast matrix mating test system or, more preferably, an improvement thereof as described in International Patent Publication No. WO 97/47763. Because the interactions are screened for in yeast, the intermolecular protein interactions detected in this system occur under physiological conditions that mimic the conditions in mammalian cells (Chien et al., 1991, Proc. Natl. Acad. Sci. USA 88:9578-9581).

Identification of interacting proteins by the improved yeast matrix mating test is based upon the detection of the expression of a reporter gene ("Reporter Gene"), the transcription of which is dependent upon the reconstitution of a transcriptional regulator by the interaction of two proteins, each fused to one half of the transcriptional regulator. The bait flt-4 or derivative, homolog or analog and prey proteins (proteins to be tested for ability to interact with the bait) are expressed as fusion proteins to a DNA binding domain, and to a transcriptional regulatory domain, respectively, or *vice versa*. In various specific embodiments, the prey has a complexity of at least 50, 100, 500, 1,000, 5,000, 10,000, or 50,000; or has a complexity in the range of 25 to 100,000,100 to 100,000, 50,000 to 100,000, or 100,000 to 500,000. For example, the prey population can be one or more nucleic acids encoding mutants of VEGF-C or VEGF-D, *e.g.*, as generated by site-directed mutagenesis or another method of making mutations in a nucleotide sequence. Preferably, the prey populations are proteins encoded by DNA, *e*.*g*., cDNA, genomic DNA, or synthetically-generated DNA. For example, the populations can be expressed from chimeric genes comprising cDNA sequences from an uncharacterized sample of a population of cDNA from mammalian RNA. Preferably, the prey population are proteins encoded by DNA, *e*.*g*., cDNA or genomic DNA or synthetically-generated DNA.

In a specific embodiment, recombinant biological libraries expressing random peptides can be used as the source of prey nucleic acids.

In another embodiment of the present invention, the invention provides a method of screening for inhibitors or enhancers of the interacting proteins identified herein. Briefly, the protein-protein interaction assay can be carried out as described herein, except that it is done in the presence of one or more candidate molecules. An increase or decrease in Reporter Gene activity relative to that present when the one or more candidate molecules are absent indicates that the candidate molecule has an effect on the interacting pair. In a preferred method, inhibition of the interaction is selected for (*i*.*e*., inhibition of the interaction is necessary for the cells to survive), for example, where the interaction activates the URA3 gene, causing yeast to die in medium containing the chemical 5-fluoroorotic acid (Rothstein, 1983, Meth. Enzymol. 101:167-180). The identification of inhibitors of such interactions can also be accomplished, for example, but not by way of limitation, using competitive inhibitor assays, as described, *supra*.

In general, proteins of the bait and prey populations are provided as fusion (chimeric) proteins (preferably by recombinant expression of a chimeric coding sequence) containing each protein contiguous to a pre-selected sequence. For one population, the pre-selected sequence is a DNA binding domain. The DNA binding domain can be any DNA binding domain, as long as it specifically recognizes a DNA sequence within a promoter, or a DNA sequence that modulates the activity of an DNA promoter, *e.g.*, an enhancer element. For example, the DNA binding domain is of a transcriptional activator or inhibitor. For the other population, the pre-selected sequence is an activator or inhibitor domain of a transcriptional activator or inhibitor, respectively. The regulatory domain alone (not as a fusion to a protein sequence) and the DNA-binding domain alone (not as a fusion to a protein sequence) preferably do not interact detectably so as to avoid false positives in the assay. The assay system further includes a reporter gene operably linked to a promoter that contains a binding site for the DNA binding domain of the transcriptional activator (or inhibitor). Accordingly, in the method of the invention, binding of a flt-4 fusion protein to a prey fusion protein leads to reconstitution of a transcriptional activator (or inhibitor) which activates (or inhibits) expression of the Reporter Gene. The activation of transcription of the Reporter Gene occurs intracellularly, *e.g.*, in prokaryotic or eukaryotic cells, preferably in cell culture.

The promoter that is operably linked to the reporter gene nucleotide sequence can be a native or non-native promoter of the nucleotide sequence, and the DNA binding site(s) that are recognized by the DNA binding domain portion of the fusion protein can be native to the promoter (if the promoter normally contains such binding site(s)) or non-native. Thus, for example, one or more tandem copies (*e.g.*, 4 or 5 copies) of the appropriate DNA binding site can be introduced upstream of the TATA box in the desired promoter (*e*.*g*., in the area of position -100 to -400). In a preferred aspect, 4 or 5 tandem copies of the 17 bp UAS (GAL4 DNA binding site) are introduced upstream of the TATA box in the desired promoter, which is upstream of the desired coding sequence for a selectable or detectable marker. In a preferred embodiment, the GAL1-10 promoter is operably fused to the desired nucleotide sequence; the GAL1-10 promoter already contains 5 binding sites for GAL4. Alternatively, the transcriptional activation binding site of the desired gene(s) can be deleted and replaced with GAL4 binding sites (Bartel et al., 1993, BioTechniques 14(6):920-924; Chasman et al., 1989, Mol. Cell. Biol. 9:4746-4749). The Reporter Gene preferably contains the sequence encoding a detectable or selectable marker the expression of which is regulated by the transcriptional activator, such that the marker is either turned on or off in the cell in response to the presence of a specific interaction. Preferably, the assay is carried out in the absence of background levels of the transcriptional activator (*e.g.*, in a cell that is mutant or otherwise lacking in the transcriptional activator). In one embodiment, more than one Reporter Gene is used to detect transcriptional activation, *e.g.*, one Reporter Gene encoding a detectable marker and one or more Reporter Genes encoding different selectable markers. The detectable marker can be any molecule that can give rise to a detectable signal, *e.g.*, a fluorescent protein or a protein that can be readily visualized or that is recognizable by a specific antibody. The selectable marker can be any protein molecule that confers ability to grow under conditions that do not support the growth of cells not expressing the selectable marker, *e.g.*, the selectable marker is an enzyme that provides an essential nutrient and the cell in which the interaction assay occurs is deficient in the enzyme and the selection medium lacks such nutrient. The Reporter Gene can either be under the control of the native promoter that naturally contains a binding site for the DNA binding protein, or under the control of a heterologous or synthetic promoter.

The activation domain and DNA binding domain used in the assay can be from a wide variety of transcriptional activator proteins, as long as these transcriptional activators have separable binding and transcriptional activation domains. For example, the Gal4 protein of *S*. *cerevisiae*, the Gcn4 protein of *S*. *cerevisiae* (Hope and Struhl, 1986, Cell 46:885-894), the Ard1 protein of *S*. *cerevisiae* (Thukral et al., 1989, Mol. Cell. Biol. 9:2360-2369), Ace1 regulatory protein of *S*. *cerevisiae* (Thiele et al., 1988, Mol. Cell. Biol. 8: 2745-2752), LexA repressor protein of *E*. *coli* (Schnarr et al., 1991, Biochimie 73:423-431), and herpesvirus VP16 transactivator (Hippenmeyer et al., 1995, Curr. Opin. Biotechnol. 6: 548-552), and the human estrogen receptor (Kumar et al., 1987, Cell 51:941-951) have separable DNA binding and activation domains. The DNA binding domain and activation domain that are employed in the fusion proteins need not be from the same transcriptional activator. In a specific embodiment, a Gal4 or LexA DNA binding domain is employed. In another specific embodiment, a Gal4 or herpes simplex virus VP16 (Triezenberg et al., 1988, Genes Dev. 2:730-742) activation domain is employed. In a specific embodiment, amino acids 1-147 of Gal4 (Ma et al., 1987, Cell 48:847-853; Ptashne et al., 1990, Nature 346:329-331) is the DNA binding domain, and amino acids 411-455 of VP16 (Triezenberg et al., 1988, Genes Dev. 2:730-742; Cress et al., 1991, Science 251:87-90) is the activation domain.

In a preferred embodiment, the yeast transcription factor Ga14 is reconstituted by the protein-protein interaction and the host strain is mutant for Ga14. In another embodiment, the DNA-binding domain is Ace1N and/or the activation domain is Ace1N, the DNA binding and activation domains of the Ace1N protein, respectively. Ace1N is a yeast protein that activates transcription from the CUP1 operon in the presence of divalent copper. CUP1 encodes metallothionein, which chelates copper, and the expression of Cup1 protein allows growth in the presence of copper, which is otherwise toxic to the host cells. The Reporter Gene can also be a CUP1-lacZ fusion that expresses the enzyme β-galactosidase (detectable by routine chromogenic assay) upon binding of a reconstituted Ace1 transcriptional activator (Chaudhuri et al., 1995, FEBS Letters 357:221-226). In another specific embodiment, the DNA binding domain of the human estrogen receptor is used, with a Reporter Gene driven by one or three estrogen receptor response elements (Le Douarin et al., 1995, Nucl. Acids. Res. 23:876-878).

The DNA binding domain and the transcription activator/inhibitor domain each preferably has a nuclear localization signal (Ylikomi et al., 1992, EMBO J. 11:3681-3694; Dingwall and Laskey, 1991, Trends Biochem. Sci. 16:479-481) functional in the cell in which the fusion proteins are to be expressed.

To facilitate isolation of the encoded proteins, the fusion constructs can further contain sequences encoding affinity tags such as glutathione-S-transferase or maltose-binding protein or an epitope of an available antibody, for affinity purification (*e.g.*, binding to glutathione, maltose, or a particular antibody specific for the epitope, respectively) (Allen et al., 1995, Trends Biochem. Sci. 20:511-516). In another embodiment, the fusion constructs further comprise bacterial promoter sequences for recombinant production of the fusion protein in bacterial cells (Allen et al., 1995, Trends Biochem. Sci. 20:511-516).

The host cell in which the interaction assay occurs can be any cell, prokaryotic or eukaryotic, in which transcription of the Reporter Gene can occur and be detected, including but not limited to mammalian (*e*.*g*., monkey, chicken, mouse, rat, human, bovine), bacterial, and insect cells, and is preferably a yeast cell. Expression constructs encoding and capable of expressing the binding domain fusion proteins, the transcriptional activation domain fusion proteins, and the Reporter Gene product(s), are provided within the host cell, by mating of cells containing the expression constructs, or by cell fusion, transformation, electroporation, microinjection, etc. In a specific embodiment in which the assay is carried out in mammalian cells, the DNA binding domain is the GAL4 DNA binding domain, the activation domain is the herpes simplex virus VP16 transcriptional activation domain, and the Reporter Gene contains the desired reporter gene coding sequence(s) operably linked to a minimal promoter element from the adenovirus E1B gene driven by several GAL4 DNA binding sites (Fearon et al., 1992, Proc. Natl. Acad. Sci. USA 89:7958-7962). The host cell used should not express an endogenous transcription factor that binds to the same DNA site as that recognized by the DNA binding domain fusion population. Also, preferably, the host cell is mutant or otherwise lacking in an endogenous, functional form of the Reporter Gene(s) used in the assay.

Various vectors and host strains for expression of the two fusion protein populations in yeast are known and can be used (see, *e.g.*, Fields et al., U.S. Patent No. 5,1468,614; Bartel et al., 1993, "Using the two-hybrid system to detect protein-protein interactions," in Cellular Interactions in Development, Hartley, ed., Practical Approach Series xviii, IRL Press at Oxford University Press, New York, NY, pp. 153-179; Fields and Sternglanz, 1994, Trends in Genetics 10:286-292). By way of example but not limitation, yeast strains or derivative strains made therefrom, which can be used are N105, N106, N1051, N1061, and YULH. Exemplary strains that can be used in the assay of the invention also include, but are not limited to, the following:
Y190: *MATa, ura3-52, his3-200, lys2-801, ade2-101, trp1-901, leu2-3,112, gal4α, gal80α, cyh*^{*r*}*2, LYS2::GALl*_{*UAS*}*-HIS3*_{*TATA*}*HIS3, URA3::GALl*_{*UAS*}*-GALl*_{*TATA*}*-lacZ* (available from Clontech, Palo Alto, CA; Harper et al., 1993, Cell 75:805-816). Y190 contains HIS3 and lacZ Reporter Genes driven by GAL4 binding sites.
CG-1945: *MATa, ura3-52, his3-200, lys2-801, ade2-101, trpl-901, leu2-3,112, gal4-542, gal80-538, cyh*^{*r*}*2, LYS2::GALl*_{*UAS*}*-HIS3*_{*TATA*}*HIS3, URA3::GALl*_{*UAS17mers(x3*}*)-CYC1*_{*TATA*}*-laCZ* (available from Clontech, Palo Alto, CA). CG-1945 contains HIS3 and lacZ Reporter Genes driven by GAL4 binding sites.
Y187: *MATα, ura3-52, his3-200, ade2-101, trp1-901, leu2-3,112, gal4a, ga180α, URA3::GAL1*_{*UAS*}*-GAL1*_{*TATA*}*-IacZ* (available from Clontech, Palo Alto, CA). Y187 contains a lacZ Reporter Gene driven by GAL4 binding sites.
SFY526: *MATa, ura3-52, his3-200, lys2-801, ade2-101, trp1-901, leu2-3,112, ga14-542, ga180-538, can*^{*r*}*, URA3::GAL1-lacZ* (available from Clontech, Palo Alto, CA). SFY526 contains HIS3 and lacZ Reporter Genes driven by GAL4 binding sites.
HF7c: *MATa, ura3-52, his3-200, lys2-801, ade2-101, trpl-901, leu2-3,112, gal4-542, gal80-538, LYS2::GAL1-HIS3, URA3::GAL1*_{*UAS17mers(x3)*}*-CYC1-lacZ* (available from Clontech, Palo Alto, CA). HF7c contains HIS3 and lacZ Reporter Genes driven by GAL4 binding sites.
YRG-2: *MATa, ura3-52, his3-200, lys2-801, ade2-101, trp1-901, leu2-3,112, gal4-542, gal80-538, LYS2::GAL1*_{*UAS*}*-GAL1*_{*TATA*}*-HIS3, URA3::GAL1*_{*UAS17mers(x3)*}*-CYC1-lacZ* (available from Stratagene Cloning Systems, La Jolla, CA). YRG-2 contains HIS3 and lacZ Reporter Genes driven by GAL4 binding sites.
Many other strains commonly known and available in the art can be used.

If not already lacking in endogenous Reporter Gene activity, cells mutant in the Reporter Gene may be selected by known methods, or the cells can be made mutant in the target Reporter Gene by known gene-disruption methods prior to introducing the Reporter Gene (Rothstein, 1983, Meth. Enzymol. 101:202-211).

In a specific embodiment, plasmids encoding the different fusion protein populations can be both introduced into a single host cell (*e.g.*, a haploid yeast cell) containing one or more Reporter Genes, by co-transformation, to conduct the assay for protein-protein interactions. More preferably, the two fusion protein populations are introduced into a single cell either by mating (*e.g.*, of yeast cells) or cell fusions (*e.g.*, of mammalian cells). In a mating type assay, conjugation of haploid yeast cells of opposite mating type that have been transformed, respectively, with a binding domain fusion expression construct (preferably a plasmid) and an activation (or inhibitor) domain fusion expression construct (preferably a plasmid), delivers both constructs into the same diploid cell. The mating type of a yeast strain may be manipulated by transformation with the HO gene (Herskowitz and Jensen, 1991, Meth. Enzymol. 194:132-146).

In a specific embodiment, a yeast interaction mating assay is employed, using two different types of host cells, strain-types a and alpha, of the yeast *Saccharomyces cerevisiae*. The host cell preferably contains at least two Reporter Genes, each with one or more binding sites for the DNA-binding domain, *e*.*g*., of a transcriptional activator. The activator domain and DNA binding domain are each parts of chimeric proteins formed from the two respective populations of proteins. One set of host cells, for example the a strain cells, contains fusions of the library of nucleotide sequences with the DNA-binding domain of a transcriptional activator, such as GAL4. The hybrid proteins expressed in this set of host cells are capable of recognizing the DNA-binding site on the Reporter Gene. The second set of yeast host cells, for example alpha strain cells, contains nucleotide sequences encoding fusions of a library of DNA sequences fused to the activation domain of a transcriptional activator.

In a specific embodiment, the fusion protein constructs are introduced into the host cell as a set of plasmids. These plasmids are preferably capable of autonomous replication in a host yeast cell and preferably can also be propagated in *E. coli*. The plasmids contain a promoter directing the transcription of the DNA binding or activation domain fusion genes, and a transcriptional termination signal. The plasmids also preferably contain a selectable marker gene, permitting selection of cells containing the plasmids. The plasmids can be single-copy or multi-copy. Single-copy yeast plasmids that have the yeast centromere may also be used to express the activation and DNA binding domain fusions (Elledge et al., 1988, Gene 70:303-312). In another embodiment, the fusion constructs are introduced directly into the yeast chromosome via homologous recombination. The homologous recombination for these purposes is mediated through yeast sequences that are not essential for vegetative growth of yeast, *e.g.*, the MER2, MER1, ZIPI, REC102, or ME14 genes.

Bacteriophage vectors can also be used to express the DNA binding domain and/or activation domain fusion proteins. Libraries can generally be prepared faster and more easily from bacteriophage vectors than from plasmid vectors.

In a specific embodiment, the invention provides a method of detecting one or more protein-protein interactions comprising (a) recombinantly expressing flt-4 or a derivative, homolog or analog thereof in a first population of yeast cells being of a first mating type and comprising a first fusion protein containing the flt-4 sequence and a DNA binding domain, wherein said first population of yeast cells contains a first nucleotide sequence operably linked to a promoter driven by one or more DNA binding sites recognized by said DNA binding domain such that an interaction of said first fusion protein with a second fusion protein, said second fusion protein comprising a transcriptional activation domain, results in increased transcription of said first nucleotide sequence; (b) negatively selecting to eliminate those yeast cells in said first population in which said increased transcription of said first nucleotide sequence occurs in the absence of said second fusion protein; (c) recombinantly expressing in a second population of yeast cells of a second mating type different from said first mating type, a plurality of said second fusion proteins, each second fusion protein comprising a sequence of a fragment, derivative, homolog or analog of VEGF-C or VEGF-D and an activation domain of a transcriptional activator, in which the activation domain is the same in each said second fusion protein; (d) mating said first population of yeast cells with said second population of yeast cells to form a third population of diploid yeast cells, wherein said third population of diploid yeast cells contains a second nucleotide sequence operably linked to a promoter driven by a DNA binding site recognized by said DNA binding domain such that an interaction of a first fusion protein with a second fusion protein results in increased transcription of said second nucleotide sequence, in which the first and second nucleotide sequences can be the same or different; and (e) detecting said increased transcription of said first and/or second nucleotide sequence, thereby detecting an interaction between a first fusion protein and a second fusion protein.

In a specific embodiment, the bait flt-4 sequence and the prey library of chimeric genes are combined by mating the two yeast strains on solid media for a period of approximately 6-8 hours. Optionally, the mating is performed in liquid media. The resulting diploids contain both kinds of chimeric genes, *i.e.*, the DNA-binding domain fusion and the activation domain fusion.

Preferred reporter genes include the URA3, HIS3 and/or the lacZ genes (*e.g.*, Rose and Botstein, 1983, Meth. Enzymol. 101:167-180) operably linked to GAL4 DNA-binding domain recognition elements. Other reporter genes comprise the functional coding sequences for, but not limited to, Green Fluorescent Protein (GFP) (Cubitt et al., 1995, Trends Biochem. Sci. 20:448-455), luciferase, LEU2, LYS2, ADE2, TRP1, CAN1, CYH2, GUS, CUP1, or chloramphenicol acetyl transferase (CAT). Expression of LEU2, LYS2, ADE2 and TRP1 are detected by growth in a specific defined media; GUS and CAT can be monitored by well known enzyme assays; and CAN1 and CYH2 are detected by selection in the presence of canavanine and cycloheximide, respectively. With respect to GFP, the natural fluorescence of the protein is detected.

In a specific embodiment, transcription of the Reporter Gene is detected by a linked replication assay. For example, as described by Vasavada et al., 1991, Proc. Natl. Acad. Sci. USA 88:10686-10690, expression of SV40 large T antigen is under the control of the E1B promoter responsive to GAL4 binding sites. The replication of a plasmid containing the SV40 origin of replication indicates the reconstruction of the GAL4 protein and a protein-protein interaction. Alternatively, a polyoma virus replicon can be employed (Vasavada et al., 1991, Proc. Natl. Acad. Sci. USA 88:10686-10690).

In another embodiment, the expression of Reporter Genes that encode proteins can be detected by immunoassay, *i*.*e*., by detecting the immunospecific binding of an antibody to such protein, which antibody can be labeled, or alternatively, which antibody can be incubated with a labeled binding partner to the antibody, so as to yield a detectable signal.

Alam and Cook, 1990, Anal. Biochem. 188:245-254 disclose non-limiting examples of detectable marker genes that can be operably linked to a transcriptional regulatory region responsive to a reconstituted transcriptional activator, and thus can be used as Reporter Genes.

The activation of Reporter Genes like URA3 or HIS3 enables the cells to grow in the absence of uracil or histidine, respectively, and hence serves as selectable markers. Thus, after mating, the cells exhibiting protein-protein interactions are selected for the ability to grow in media lacking a nutritional component, such as uracil or histidine, respectively (referred to as -URA (URA minus) and -HIS (HIS minus) medium, respectively). The -HIS medium preferably contains 3-amino-1,2,4-triazole (3-AT), which is a competitive inhibitor of the HIS3 gene product and thus requires higher levels of transcription to achieve selection (Durfee et al., 1993, Genes Dev. 7:555-569). Similarly, 6-azauracil, which is an inhibitor of the URA3 gene product, can be included in -URA medium (Le Douarin et al., 1995, Nucl. Acids Res. 23:876-878). URA3 gene activity can also be detected and/or measured by determining the activity of its gene product, orotidine-5'-monophosphate decarboxylase (Pierrat et al., 1992, Gene 119:237-245; Wolcott et al., 1966, Biochem. Biophys. Acta 122:532-534). In other embodiments of the invention, the activities of the reporter genes like lacZ or GFP are monitored by measuring a detectable signal (*e.g.*, chromogenic or fluorescent, respectively) that results from the activation of these Reporter Genes. For example, lacZ transcription can be monitored by incubation in the presence of a chromogenic substrate, such as X-gal (5-bromo-4-chloro-3-indolyl-β-D-galactoside), for its encoded enzyme, β-galactosidase. The pool of all interacting proteins isolated in this manner by mating the flt-4 sequence product and the library identifies the "flt-4 interactive population".

In a specific embodiment of the invention, false positives arising from transcriptional activation by the DNA binding domain fusion proteins in the absence of a transcriptional activator domain fusion protein are prevented or reduced by negative selection for such activation within a host cell containing the DNA binding fusion population, prior to exposure to the activation domain fusion population. By way of example, if such cell contains URA3 as a Reporter Gene, negative selection is carried out by incubating the cell in the presence of 5-fluoroorotic acid (5-FOA) which kills URA+ cells (Rothstein, 1983, Meth. Enzymol. 101:167-180). Hence, if the DNA-binding domain fusions by themselves activate transcription, the metabolism of 5-FOA will lead to cell death and the removal of self-activating DNA-binding domain hybrids.

Negative selection involving the use of a selectable marker as a Reporter Gene and the presence in the cell medium of an agent toxic or growth inhibitory to the host cells in the absence of Reporter Gene transcription is preferred, since it allows a higher rate of processing than other methods. As will be apparent, negative selection can also be carried out on the activation domain fusion population prior to interaction with the DNA binding domain fusion population, by similar methods, either alone or in addition to negative selection of the DNA binding fusion population.

Negative selection can also be carried out on the recovered flt-4:VEGF-C/D pairs by known methods (*e*.*g*., Bartel et al., 1993, BioTechniques 14:920-924) although pre-negative selection (prior to the interaction assay), as described above, is preferred. For example, each plasmid encoding a protein or peptide or polypeptide fused to the activation domain (one-half of a detected interacting pair) can be transformed back into the original screening strain, either alone or with a plasmid encoding only the DNA-binding domain, the DNA-binding domain fused to the detected interacting protein, or the DNA-binding domain fused to a protein that does not affect transcription or participate in the protein-protein interaction; a positive interaction detected with any plasmid other than that encoding the DNA-binding domain fusion to the detected interacting protein indicates that the activation domain yields false positives, and it is subsequently eliminated from the screen.

In a specific embodiment, the flt-4 plasmid population is transformed in a yeast strain of a first mating type (a or alpha), and the second plasmid population (containing the library of DNA sequences) is transformed in a yeast strain of different mating type. Both strains are preferably mutant for URA3 and HIS3, and contain URA3, and optionally lacZ, as a Reporter Genes. The first set of yeast cells are positively selected for the flt-4 plasmids and are negatively selected for false positives by incubation in medium lacking the selectable marker (*e.g.*, uracil) and containing 5-FOA. Yeast cells of the second mating type are transformed with the second plasmid population, and are positively selected for the presence of the plasmids containing the library of fusion proteins. Selected cells are pooled. Both groups of pooled cells are mixed together and mating is allowed to occur on a solid phase. The resulting diploid cells are then transferred to selective media that selects for the presence of each plasmid and for activation of Reporter Genes.

In a specific embodiment of the invention, after an interactive population is obtained, the DNA sequences encoding the pairs of interactive proteins are isolated by a method wherein either the DNA-binding domain hybrids or the activation domain hybrids are amplified, in separate respective reactions. Preferably, the amplification is carried out by polymerase chain reaction (PCR) (U.S. Patent Nos. 4,683,202, 4,683,195 and 4,889,818; Gyllenstein et al., 1988, Proc. Natl. Acad. Sci. USA 85:7652-7656; Ochman et al., 1988, Genetics 120:621-623; Loh et al., 1989, Science 243:217-220; Innis et al., 1990, PCR Protocols, Academic Press, Inc., San Diego, CA), using pairs of oligonucleotide primers specific for either the DNA-binding domain hybrids or the activation domain hybrids. This PCR reaction can also be performed on pooled cells expressing interacting protein pairs, preferably as pooled arrays of interactants. Other amplification methods known in the art can be used, including but not limited to ligase chain reaction (EP 320,308), use of Qβ replicase, or methods listed in Kricka et al., 1995, Molecular Probing, Blotting, and Sequencing, Chap. 1 and Table IX, Academic Press, New York.

The plasmids encoding the DNA-binding domain hybrid proteins and the activation domain hybrid proteins can also be isolated and cloned by any of the methods well known in the art. For example, but not by way of limitation, if a shuttle (yeast to *E. coli*) vector is used to express the fusion proteins, the genes can be recovered by transforming the yeast DNA into *E. coli* and recovering the plasmids from *E. coli* (*e.g.,* Hoffman et al., 1987, Gene 57:267-272). Alternatively, the yeast vector can be isolated, and the insert encoding the fusion protein subcloned into a bacterial expression vector, for growth of the plasmid in *E. coli*.

### 5.5. PHARMACEUTICAL COMPOSITIONS AND THERAPEUTIC/PROPHYLACTIC ADMINISTRATION

The invention provides methods of treatment (and prophylaxis) by administration to a subject of an effective amount of a Therapeutic of the invention. In a preferred aspect, the Therapeutic is substantially purified. The subject is preferably an animal including, but not limited to animals such as cows, pigs, horses, chickens, cats, dogs, etc., and is preferably a mammal, and most preferably human. In a specific embodiment, a non-human mammal is the subject.

Formulations and methods of administration that can be employed when the Therapeutic comprises a nucleic acid are described in Sections 5.2.1 and 5.2.2, *supra*; additional appropriate formulations and routes of administration can be selected from among those described herein below.

Various delivery systems are known and can be used to administer a Therapeutic of the invention, *e.g.*, encapsulation in liposomes, microparticles, and microcapsules: use of recombinant cells capable of expressing the Therapeutic, use of receptor-mediated endocytosis (*e*.*g*., Wu and Wu, 1987, J. Biol. Chem. 262:4429-4432); construction of a Therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (*e.g.*, oral, rectal and intestinal mucosa, etc.), and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g.*, by use of an inhaler or nebulizer, and formulation with an aerosolizing agent.

In a specific embodiment, it may be desirable to administer the pharmaceutical compositions of the invention locally to the area in need of treatment. This may be achieved by, for example, and not by way of limitation, local infusion during surgery, topical application, *e.g.*, in conjunction with a wound dressing after surgery, by injection, by means of a catheter, by means of a suppository, or by means of an implant, said implant being of a porous, non-porous, or gelatinous material, including membranes, such as sialastic membranes, or fibers. In one embodiment, administration can be by direct injection at the site (or former site) of a malignant tumor or neoplastic or pre-neoplastic tissue.

In another embodiment, the Therapeutic can be delivered in a vesicle, in particular a liposome (Langer, 1990, Science 249:1527-1533; Treat et al., 1989, In: Liposomes in the Therapy of Infectious Disease and Cancer, Lopez-Berestein and Fidler, eds., Liss, New York, pp. 353-365; Lopez-Berestein, ibid., pp. 317-327; see generally ibid.)

In yet another embodiment, the Therapeutic can be delivered via a controlled release system. In one embodiment, a pump may be used (Langer, *supra*; Sefton, 1987, CRC Crit. Ref. Biomed. Eng. 14:201-240; Buchwald et al., 1980, Surgery 88:507-516; Saudek et al., 1989, N. Engl. J. Med. 321:574-579). In another embodiment, polymeric materials can be used (Medical Applications of Controlled Release, Langer and Wise, eds., CRC Press, Boca Raton, Florida, 1974; Controlled Drug Bioavailability, Drug Product Design and Performance, Smolen and Ball, eds., Wiley, New York, 1984; Ranger and Peppas, 1983, Macromol. Sci. Rev. Macromol. Chem. 23:61; Levy et al., 1985, Science 228:190-192; During et al., 1989, Ann. Neurol. 25:351-356; Howard et al., 1989, J. Neurosurg. 71:858-863). In yet another embodiment, a controlled release system can be placed in proximity of the therapeutic target, *i*.*e*., the brain, thus requiring only a fraction of the systemic dose (*e.g.*, Goodson, 1984, In: Medical Applications of Controlled Release, supra, Vol. 2, pp. 115-138). Other controlled release systems are discussed in the review by Langer (1990, Science 249:1527-1533).

In a specific embodiment where the Therapeutic is a nucleic acid encoding a protein Therapeutic, the nucleic acid can be administered in vivo to promote expression of its encoded protein, by constructing it as part of an appropriate nucleic acid expression vector and administering it so that it becomes intracellular, *e.g.*, by use of a retroviral vector (U.S. Patent No. 4,980,286), or by direct injection, or by use of microparticle bombardment (*e*.*g*., a gene gun; Biolistic, Dupont), or by coating it with lipids, cell-surface receptors or transfecting agents, or by administering it in linkage to a homeobox-like peptide which is known to enter the nucleus (*e*.*g*., Joliot et al., 1991, Proc. Natl. Acad. Sci. USA 88:1864-1868), etc. Alternatively, a nucleic acid Therapeutic can be introduced intracellularly and incorporated by homologous recombination within host cell DNA for expression.

The present invention also provides pharmaceutical compositions. Such compositions comprise a therapeutically effective amount of a Therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the Therapeutic, preferably in purified form, together with a suitable amount of carrier so as to provide the form for proper administration to the patient. The formulation should suit the mode of administration.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The Therapeutics of the invention can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc.

The amount of the Therapeutic of the invention which will be effective in the treatment of a particular disorder or condition will depend on the nature of the disorder or condition, and can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from *in vitro* or animal model test systems.

Suppositories generally contain active ingredient in the range of 0.5% to 10% by weight; oral formulations preferably contain 10% to 95% active ingredient.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

### 5.6. ANIMAL MODELS

The present invention also provides animal models. In one embodiment, animal models for diseases and disorders involving flt-4:VEGF-C/D complexes are provided. These include, but are not limited to, secondary prostate tumor metastases. Such animals can be initially produced by promoting homologous recombination or insertional mutagenesis between flt-4 and VEGF-C or VEGF-D genes in the chromosome, and exogenous flt-4 and VEGF-C or VEGF-D genes that have been rendered biologically inactive or deleted (preferably by insertion of a heterologous sequence, *e.g.*, an antibiotic resistance gene). In a preferred aspect, homologous recombination is carried out by transforming embryo-derived stem (ES) cells with a vector containing the insertionally inactivated flt-4 and VEGF-C or VEGF-D gene, such that homologous recombination occurs, followed by injecting the transformed ES cells into a blastocyst, and implanting the blastocyst into a foster mother, followed by the birth of the chimeric animal ("knockout animal") in which a flt-4 and/or VEGF-C or VEGF-D gene has been inactivated or deleted (Capecchi, 1989, Science 244:1288-1292). The chimeric animal can be bred to produce additional knockout animals. Such animals can be mice, hamsters, sheep, pigs, cattle, etc., and are preferably non-human mammals. In a specific embodiment, a knockout mouse is produced.

In a different embodiment of the invention, transgenic animals that have incorporated and express (or overexpress or mis-express) a functional flt-4 and/or VEGF-C or VEGF-D gene, *e.g.* by introducing the flt-4 and VEGF-C or VEGF-D genes under the control of a heterologous promoter (*i.e.*, a promoter that is not the native flt-4 or VEGF-C or VEGF-D promoter) that either overexpresses the protein or proteins, or expresses them in tissues not normally expressing the complexes or proteins, can have use as animal models of diseases and disorders characterized by elevated levels of flt-4:VEGF-C/D complexes. Such animals can be used to screen or test molecules for the ability to treat or prevent the diseases and disorders cited *supra*.

In one embodiment, the invention provides a recombinant non-human animal containing both a flt-4 gene and a VEGF-C or VEGF-D gene in which the flt-4 gene is under the control of a promoter that is not the native flt-4 gene promoter and the VEGF-C or VEGF-D gene is under the control of a promoter that is not the native VEGF-C or VEGF-D gene promoter.

The following series of examples are presented by way of illustration and not by way of limitation on the scope of the present invention.

### 6. EXAMPLES

The following experiments demonstrate that flt-4 is expressed only in prostate cancer cells that have metastatic potential or are or are derived from secondary prostate tumor metastases of a primary prostate tumor, such that flt-4 expression in a prostate cancer cell can serve as a diagnostic and prognostic marker of prostate cancer, and further that methods inhibiting expression or activity of flt-4 are useful in the treatment, inhibition or prevention of prostate cancer.

### 6.1. FLT-4 EXPRESSION DETERMINED BY IMMUNOHISTOCHEMISTRY

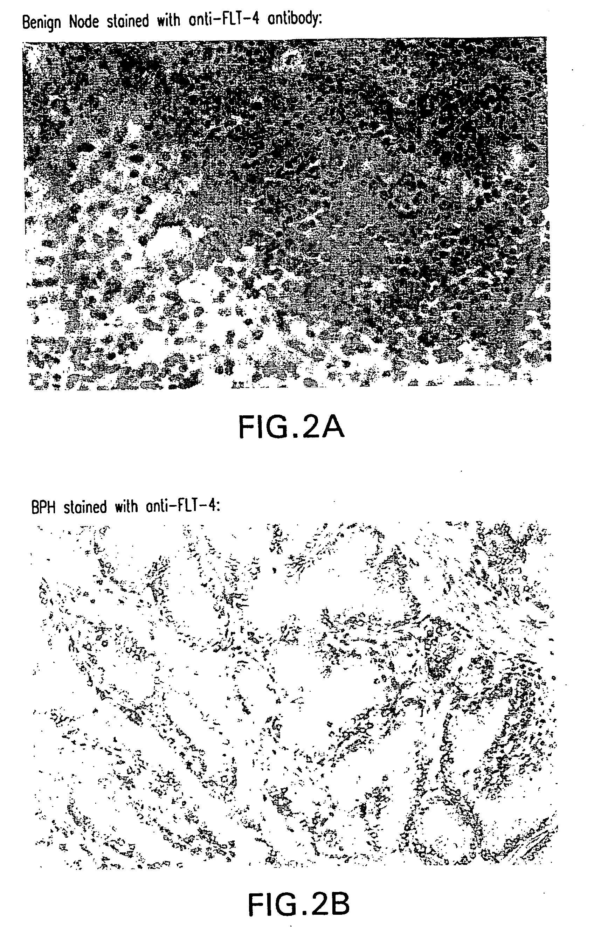

Representative tissue samples of benign prostate hyperplasia, primary prostate carcinoma, normal lymph nodes and secondary prostate tumor lymphatic metastasis were stained with an anti-flt-4 antibody (Santa Cruz Corp., Santa Cruz, CA) using the Zemed Histostain kit obtained from Zemed (So. San Francisco, CA). The immunohistochemical assay was carried out by sequential application of the diluted primary antibody for 30 minutes, biotinylated secondary antibody for 15 minutes and HRP-Streptavidin for 15 minutes. Immunoreactivity was visualized with either AEC (3-amino-9-ethylcarbazole) or DAB (3,3'-diaminobenzidine) chromogens and sections were counterstained with hematoxylin. Appropriate negative controls were performed using rabbit or goat or isotyped matched mouse antibodies and all negative controls showed very low background. Positive and negative controls were run in parallel with each batch. The results are presented in Figs. 2A-2F.

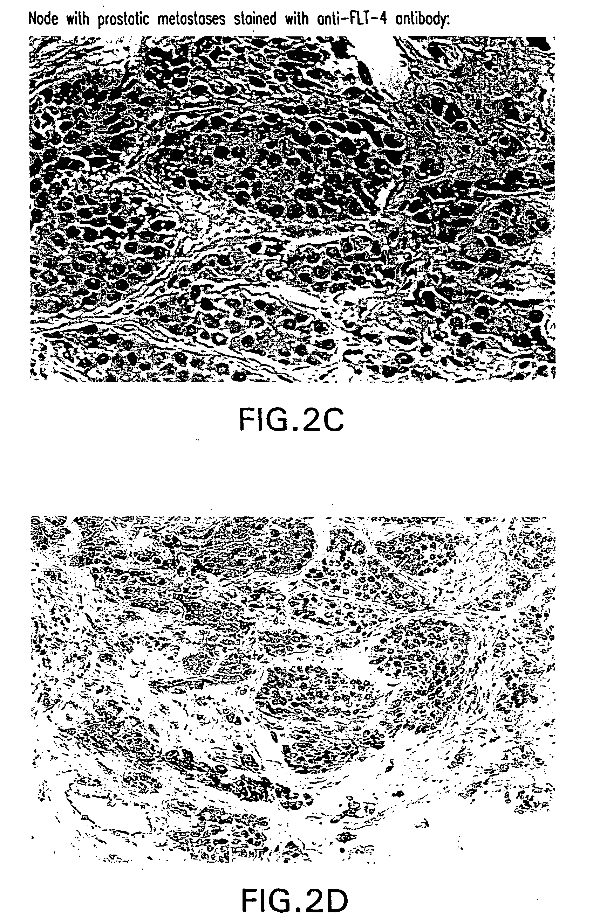

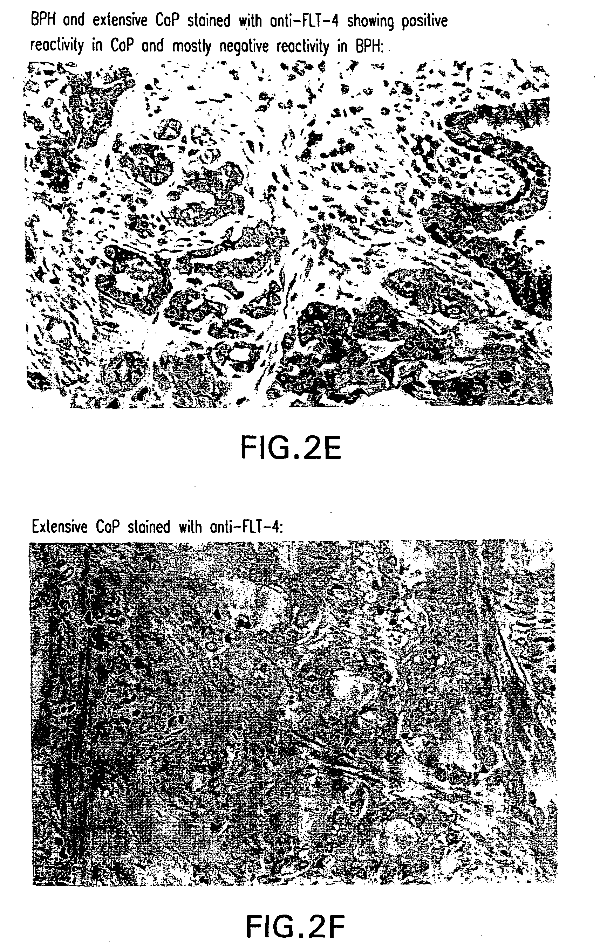

Figs. 2A and 2B, normal lymph node tissue and benign prostate hyperplasia tissue, respectively, no flt-4 expression is seen. However, flt-4 expression is evident in lymph node tissue with prostatic metastases (Figs. 2C and 2D). Fig. 2E clearly shows the difference in flt-4 expression between prostate cancer tissue and benign prostate hyperplasia tissue. Fig. 2F also clearly demonstrates that flt-4 is expressed in prostate cancer tissue.

In the clinical samples tested, all benign epithelium tested (5/5) negative for flt-4 expression. All benign lymph nodes (7/7) also tested negative for flt-4 expression. Most prostate carcinoma (9/10) also tested negative for flt-4 expression. Interestingly, the only exception for prostate carcinoma was obtained from a patient who developed local recurring disease subsequent to radical prostatectomy. All prostate cancer metastases in lymph nodes tested (2/2) positive for flt-4 expression. These same samples all tested positive for VEGF-C expression, indicating that the secondary prostate tumor metastases exhibit a VEGF-C/flt-4 autocrine loop.

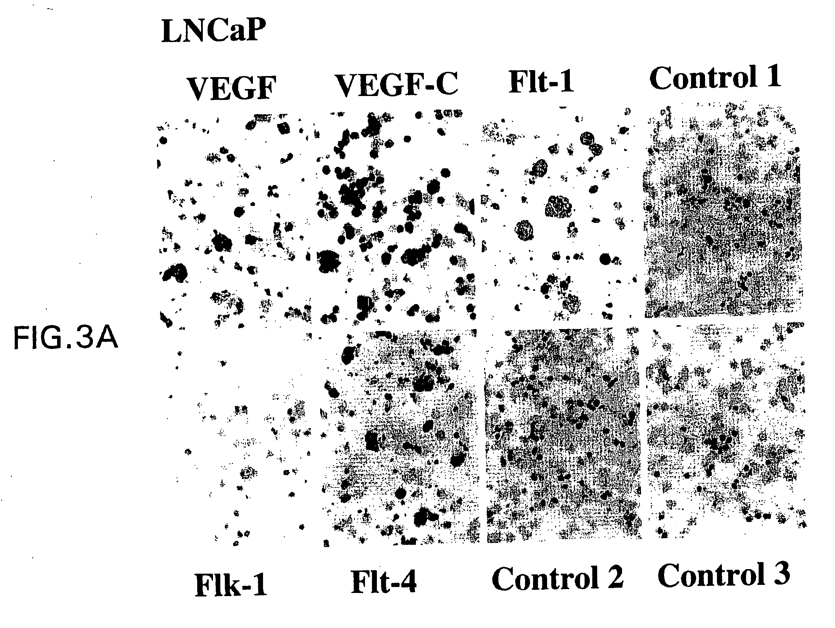

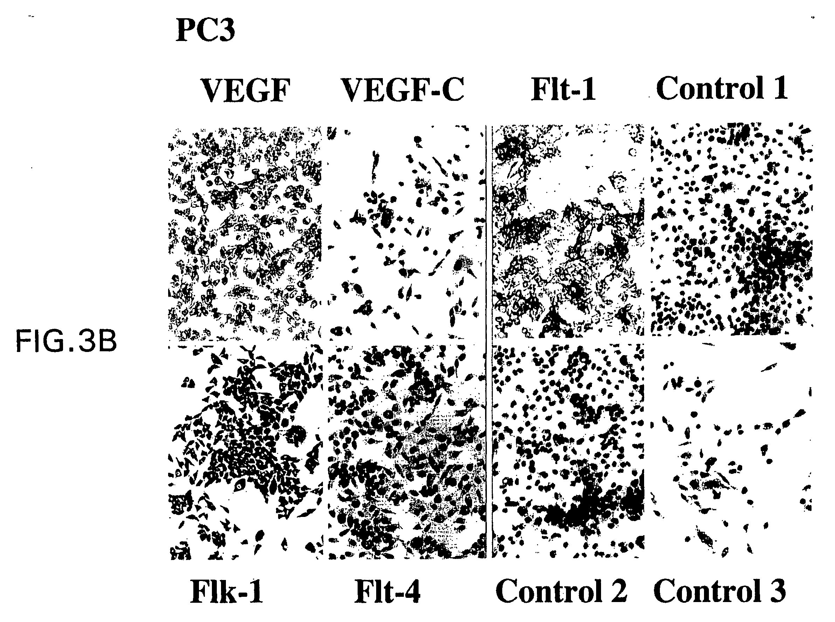

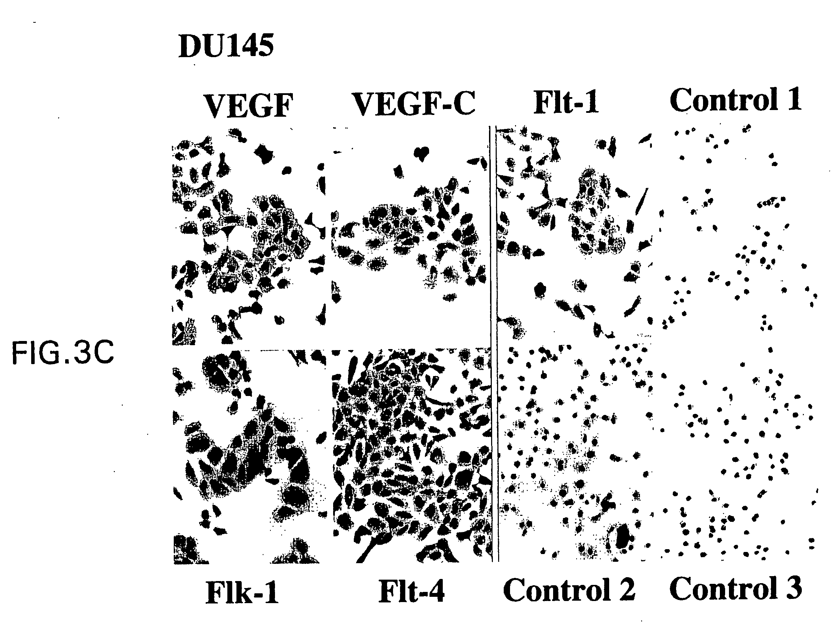

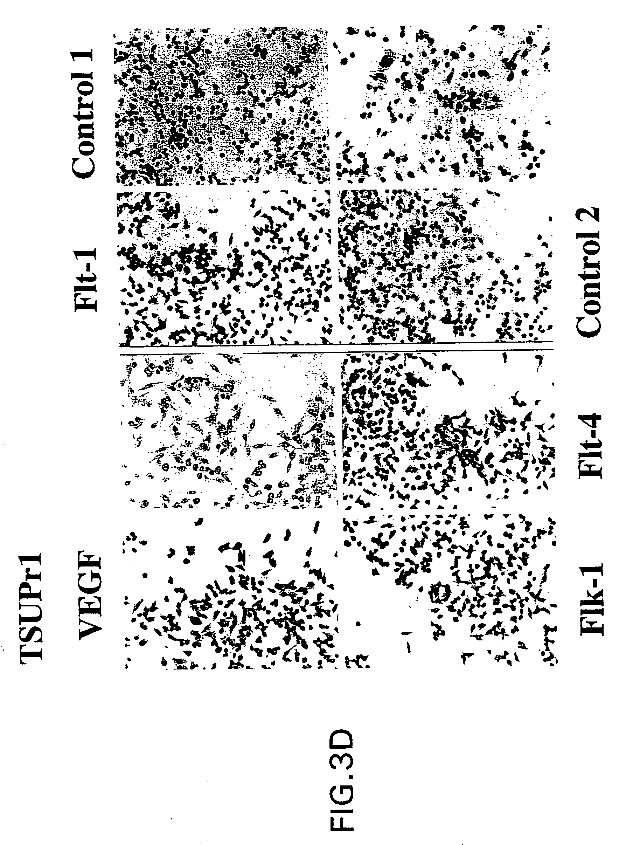

Human prostate cancer cell lines were also tested for flt-4, VEGF-C, flk-1 and VEGF expression by immunocytochemistry. The human prostate cancer cell lines, LNCaP, PC-3, DU145, and TSUPr1, were grown in a slide chamber and were fixed with 10% formalin and stained with anti-flt-4 antibody as described above. The antibodies to VEGF-C, VEGF and flk-1 were also obtained from Santa Cruz Corp. (Santa Cruz, CA). The results, that flt-4 is expressed in each human prostate cancer cell line, are shown in Figs. 3A-3D.

### 6.2. FLT-4 EXPRESSION DETERMINED BY REVERSE TRANSCRIPTASE PCR

Total RNA was isolated from human prostate cancer cell lines LNCaP, a hormone sensitive cell line, PC-3, DU-145 and TSUPr1, hormone independent cell lines, using a RNAzo1 B kit (Biotex, Houston, TX). After RNase-free DNase I treatment (Promega, Madison, WI), 5 µg of RNA was reverse transcribed using Superscript II (Gibco-BRL, London, UK).

Polymerase chain reaction ("PCR") was performed on the reverse transcribed cDNA samples using primers for VEGF and its receptor flk-1 and VEGF-C and its receptor flt-4. The primers used are as follows:

VEGF: 5'-CGAAGTGGTGAAGTTCATGGATG-3' (SEQ ID NO:4) and 5'-
TTCTGTATCAGTCTTTCCTGGTGAG-3' (SEQ ID NO:5),

flk-1: 5'-CTGGCATGGTCTTCTGTGAAAGCA-3' (SEQ ID NO:6) and 5'-
AATACCAGTGGATGTGATGCGG-3' (SEQ ID NO:7),

VEGF-C: 5'-TACCACAGTGTCAGGCAGCG-3' (SEQ ID NO:8) and 5'-
ATCAAATTCTCGGTTGGCCC-3', (SEQ ID NO:9),

flt-4: 5'-AGAGGGATGGAGTTCCTGGC-3' (SEQ ID NO:10) and 5'-
AATACCAGTGGATGTGATGCGG-3' (SEQ ID NO:11).

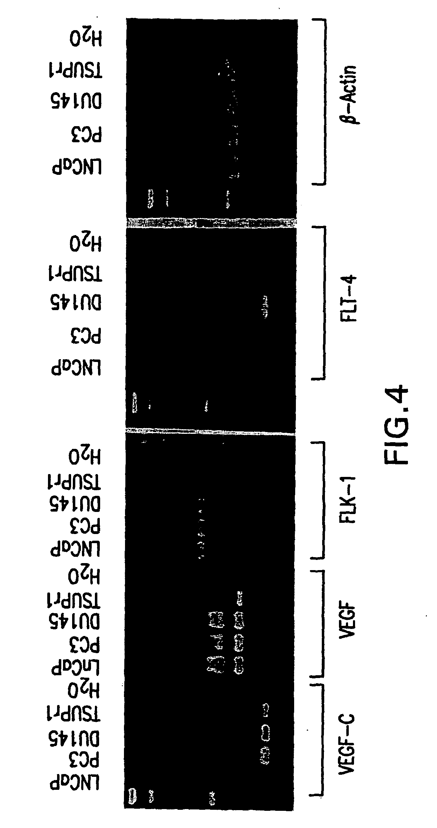

PCR was performed using Biolase *Taq* polymerase (Bioline, London, UK) with the supplied buffer and 3 mM MgCl₂ for VEGF, VEGF-C and flt-4 or with the supplied buffer and 3.5 mM MgCl₂ for flk-1. The amplification conditions were 35 cycles of denaturation at 94°C for 30 seconds; annealing at 63°C for VEGF, 62°C for flk-1, 57°C for VEGF-C and 63°C for flt-4 for 1 minute; and extension at 72°C for 1 minute. After the 35 cycles, a final extension step at 72°C for 10 minutes is performed. The amplification products were separated on 2.5% agarose gels. The results are shown in Fig. 4.

As shown in Fig. 4, flt-4 is expressed in all the human prostate cancer cell lines tested. In addition, the ligand for flt-4, VEGF-C, is also expressed in all the hormone independent human prostate cancer cell lines tested. These results agree with the immunohistochemistry results and is consistent with the presence of a VEGF-C/flt-4 autocrine loop in metastatic prostate cancer cells.

### 6.3. FLOW CYTOMETRY

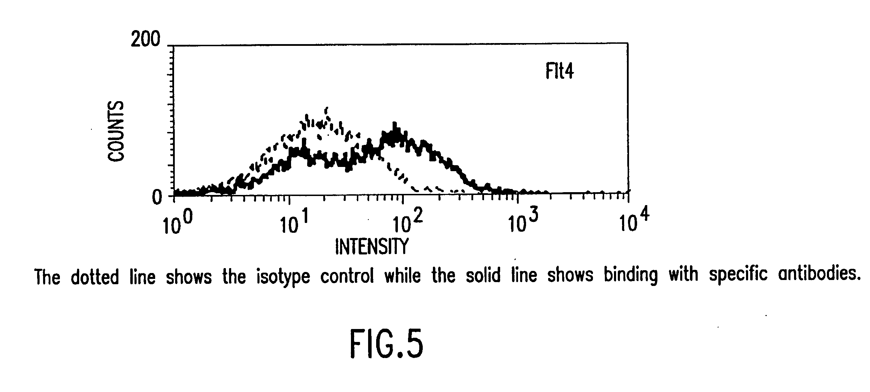

LNCaP cells (1 x 10⁶) were pelleted and incubated with rabbit anti-flt-4 serum or normal rabbit serum (negative control), each obtained from Santa Cruz Corp. (Santa Cruz, CA), for 30 minutes at 4C. Cells were washed once with PBS and then incubated with biotinylated anti-rabbit antibody for 30 minutes at 4C. After another wash with PBS, cells were incubated with strep-PE for 30 minutes at 4C. Cells were washed once more with PBS and analyzed on a Becton-Dickinson FACSCalibur flow cytometer. The LNCaP cell line was derived from a secondary prostate metastasis in lymph node tissue. The results are shown in Fig. 5.

Fig. 5 clearly shows that flt-4 expression can be detected on the surface of live LNCaP cells.

### 6.4. INHIBITION OF PC-3 GROWTH USING ANTISENSE OLIGONUCLEOTIDES

This example demonstrates that antisense oligonucleotides for inhibiting the expression of VEGF and its receptors flk-1 and flt-1 and VEGF-C and its receptor flt-4 inhibited the growth of the hormone independent human prostate cancer cell line PC-3, a cell line derived from a prostate tumor metastasis.

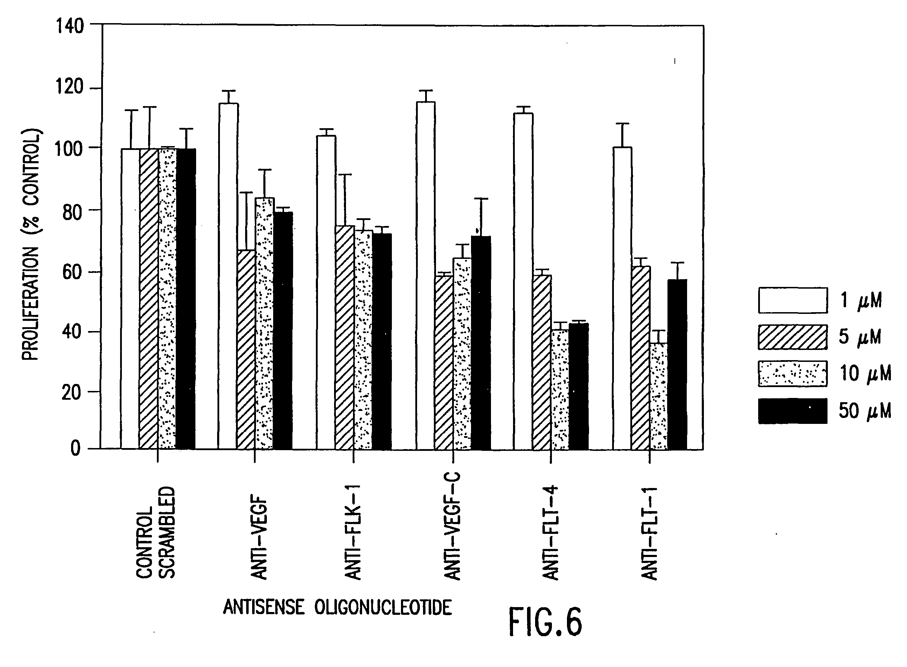

PC-3 cells were seeded at 2000 cells/well in a 96 well plate or seeded at 20,000 cells/well in a 24 well plate in serum free X-VIVO medium (Biowhittaker, Walkerville, MD). Antisense oligos were added to the cells at at increasing concentration of 1 to 100 µM. Media and oligos were refreshed every third day and the cultures were maintained for 14 days. Viable cells were quantitated either by hemacytometer cell-counting or by a MTS 96-well viable cell assay obtained from Promega (Madison, WI). Cell proliferation using the MTS assay was expressed as a percentage of control. The experiments were done in duplicate. The sequences of the oligonucleotides were as follows: Anti-VEGF: 5'-AGACAGCAGAAAGTTCATGGT-3' (SEQ ID NO:12) Anti-VEGF-C: 5'-CAAGTGCATGGTGGA-3' (SEQ ID NO:13) Anti-flk-1: 5'-CACCTTGCTCTGCAT-3' (SEQ ID NO:14) Anti-flt-1: 5'-CCCGGTGTCCCAGA-3' (SEQ ID NO:15) Anti-flt-4: 5'-GGCGCCCGCTGCAT-3' (SEQ ID NO:3) Control oligo: 5'-TACGTAGTATGGTGTAC-3' (SEQ ID NO:16) The results are shown in Fig. 6.

An oligo concentration of 100 µM proved to be toxic to the cells and is excluded from Fig. 6. As indicated in Fig. 6, growth inhibition was generally maximal at 5-10 µM. Repression of flt-1 and flt-4 expression had the greatest inhibitory effect on PC-3 cell growth, approximately 50%, followed by VEGF-C, approximately 20-30%. Anti-VEGF and anti-flk-1oligos inhibited growth by approximately 20% as compared to control oligo. The differential inhibition between VEGF-C/flt-4 and VEGF/flk-1 may reflect the relative importance of each autocrine pathway. Thus, these results demonstrate that anti-flt-4 and anti-VEGF-C antisense oligonucleotides are effective for inhibiting or suppressing prostate cancer metastases.

The invention claimed and described herein is not to be limited in scope by the specific embodiments herein disclosed since these embodiments are intended as illustrations of several aspects of the invention. Indeed, various modifications of the invention in addition to those shown and described herein will become apparent to those skilled in the art from the foregoing description. Such modifications are also intended to fall within the scope of the appended claims.

A number of references are cited herein, the entire disclosures of which are incorporated herein, in their entirety, by reference.

Preferred embodiments of the present invention will now be presented as numbered clauses
1. A method for detection of metastatic potential comprising detecting expression of flt-4 in a prostate cell, wherein expression of flt-4 indicates that said cell has metastatic potential.
2. A method for detection of metastatic potential comprising identifying a prostate cell in a body fluid sample obtained from a subject and detecting expression of flt-4 in said cell, wherein expression of flt-4 indicates that said cell is a prostate cancer cell that has metastatic potential or is a secondary prostate tumor metastasis, or is derived therefrom.
3. The method according to clause 1 or 2 in which the prostate cell is identified by using an antibody or a portion thereof that binds to a prostate cell-specific marker.
4. The method according to clause 3 in which the prostate cell-specific marker is selected from the group consisting of prostate-specific antigen (PSA), prostate-specific membrane antigen (PSMA), prostate secretory protein (PSP), prostate acid phosphatase (PAP), human glandular kallekrein 2 (HK-2), prostate stem cell antigen (PSCA) and PTI-1.
5. The method according to clause 1 or 2 in which flt-4 expression is detected using an antibody or a portion thereof that binds to flt-4.
6. The method according to clause 1 or 2 in which flt-4 expression is detected using a nucleic acid molecule, said molecule comprising a nucleotide sequence consisting of a sequence of at least 6 contiguous nucleotides complementary to the nucleotide sequence set forth in SEQ ID NO: 1.
7. The method according to clause 2 in which the body fluid is blood, urine or semen.
8. The method according to clause 2 in which identifying the prostate cell and detecting flt-4 expression are performed simultaneously.
7. The method according to clause 2 in which the body fluid is blood, urine or semen.
8. The method according to clause 2 in which identifying the prostate cell and detecting flt-4 expression are performed simultaneously.
9. The method according to clause 8 in which any immunofluorescence assay is employed.
10. The method according to clause 9 in which the immunofluorescence assay employs a flow cytometer or a laser scanning cytometer.
11. A method for diagnosing metastatic prostate cancer in a subject comprising identifying a prostate cell in a body fluid sample obtained from the subject and detecting expression of flt-4 in the prostate cell, wherein expression of flt-4 in a prostate cell indicates that the subject has metastatic prostate cancer.
12. A method for determining the prognosis of a subject with prostate cancer comprising identifying a prostate cell in a body fluid sample obtained from a subject with prostate cancer and detecting expression of flt-4 in the prostate cell, wherein expression of fit-4 in said cell indicates that the subject has a worse prognosis as compared to a second subject in whose prostate cell no flt-4 expression or activity is detected.
13. A method of treating, inhibiting or preventing a secondary prostate tumor metastasis comprising administering to a subject in which such treatment, inhibition or prevention is desired a therapeutically effective amount of a molecule that inhibits fit-4 expression or activity.
14. The method according to clause 13 in which the molecule is a protein comprising a fragment of flt-4, which fragment consists of at least the amino acid sequence set forth in SEQ ID NO:2, which protein acts as a competitive inhibitor of fit-4 binding to its ligand VEGF-C.
15. The method according to clause 14 in which the protein is soluble.
16. The method according to clause 13 in which the molecule is a nucleic acid molecule comprising a nucleotide sequence which encodes for a fragment of fit-4, which fragment acts as a competitive inhibitor of flt-4 binding to its ligand VEGF-C.
17. The method according to clause 13 in which the molecule is an antisense oligonucleotide comprising a nucleotide sequence consisting of at least 6 contiguous nucleotides complementary to the nucleotide sequence set forth in SEQ ID NO: 1.
18. The method according to clause 17 in which the antisense oligonucleotide comprises the nucleotide sequence 5'-GGCGCCCCGCTGCAT-3' (SEQ ID NO:3).
19. The method according to clause 13 in which the molecule is an antibody or a portion thereof that binds to flt-4.
20. A method for screening for a molecule that treats, inhibits or prevents a secondary prostate tumor metastasis comprising contacting a prostate cell that expresses flt-4 with a candidate molecule and comparing the level of flt-4 expression in the cell so contacted with a prostate cell expressing fit-4 not so contacted, wherein a lower level of fit-4 expression in the contacted cell as compared to the non-contacted cell indicates that the candidate molecule has activity in treating, inhibiting or preventing secondary prostate tumor metastases.
21. A method for screening for a molecule that treats, inhibits or prevents a secondary prostate tumor metastasis comprising measuring the levels of complex formed from fit-4 and VEGF-C in the presence of a candidate molecule under conditions conducive to the formation of said complex; and comparing levels of said complex that are formed in the absence of the molecule, wherein a lower level of said complex in the presence of the molecule indicates that the candidate molecule has activity in treating, inhibiting or preventing secondary prostate tumor metastases.
22. A method for screening for a molecule that treats, inhibits or prevents a secondary prostate tumor metastasis comprising measuring the levels of complex formed from flt-4 and VEGF-D in the presence of a candidate molecule under conditions conducive to the formation of said complex; and comparing levels of said complex that are formed in the absence of the molecule, wherein a lower level of said complex in the presence of the molecule indicates that the candidate molecule has activity in treating, inhibiting or preventing secondary prostate tumor metastases.
23. A method of monitoring the efficacy of a method of treatment or inhibition of metastatic prostate cancer comprising measuring the level of expression or activity of flt-4 in prostate cells obtained from a subject wherein said sample is taken from said subject after the application of said method and compared to (a) said level in a sample taken from said subject prior to the application of said method or (b) a standard level associated with the pretreatment stage of metastatic prostate cancer, in which a decrease in the level of flt-4 expression or activity in said sample taken after application of said method relative to the level of flt-4 expression or activity in said sample taken before application of said method or to said standard level indicates that said method is effective.
24. A pharmaceutical composition comprising a protein, which protein comprises a fragment of flt-4, which fragment consists of at least the amino acid sequence set forth in SEQ ID NO:2, which protein acts as a competitive inhibitor of flt-4 binding to its ligand VEGF-C; and a pharmaceutically acceptable carrier.
25. The composition according to clause 24 in which the protein is soluble.
26. A pharmaceutical composition comprising a nucleic acid, which nucleic acid comprises a nucleotide sequence which encodes for a fragment of flt-4, which fragment acts as a competitive inhibitor of flt-4 binding to its ligand VEGF-C; and a pharmaceutically acceptable carrier.
27. A pharmaceutical composition comprising an antisense oligonucleotide, which oligonucleotide comprises a nucleotide sequence consisting of at least 6 contiguous nucleotides complementary to the nucleotide sequence set forth in SEQ ID NO: 1; and a pharmaceutically acceptable carrier.
28. The composition according to clause 27 in which the antisense oligonucleotide comprises the nucleotide sequence 5'-GGCGCCCCGCTGCAT-3' (SEQ ID NO:3).
29. A pharmaceutical composition comprising an antibody or a portion thereof that binds to flt-4; and a pharmaceutically acceptable carrier.

## Claims

1. A method of treating, inhibiting or preventing a secondary prostate tumor metastasis comprising administering to a subject in which such treatment, inhibition or prevention is desired a therapeutically effective amount of a molecule that inhibits fit-4 expression or activity.

2. The method according to claim 1 in which the molecule is a protein comprising a fragment of flt-4, which fragment consists of at least the amino acid sequence set forth in SEQ ID NO:2, which protein acts as a competitive inhibitor of fit-4 binding to its ligand VEGF-C.

3. The method according to claim 2 in which the protein is soluble.

4. The method according to claim 1 in which the molecule is a nucleic acid molecule comprising a nucleotide sequence which encodes for a fragment of fit-4, which fragment acts as a competitive inhibitor of flt-4 binding to its ligand VEGF-C.

5. The method according to claim 1 in which the molecule is an antisense oligonucleotide comprising a nucleotide sequence consisting of at least 6 contiguous nucleotides complementary to the nucleotide sequence set forth in SEQ ID NO: 1.

6. The method according to claim 5 in which the antisense oligonucleotide comprises the nucleotide sequence 5'-GGCGCCCCGCTGCAT-3' (SEQ ID NO:3).

7. The method according to claim 1 in which the molecule is an antibody or a portion thereof that binds to flt-4.

8. A method for screening for a molecule that treats, inhibits or prevents a secondary prostate tumor metastasis comprising contacting a prostate cell that expresses flt-4 with a candidate molecule and comparing the level of flt-4 expression in the cell so contacted with a prostate cell expressing fit-4 not so contacted, wherein a lower level of fit-4 expression in the contacted cell as compared to the non-contacted cell indicates that the candidate molecule has activity in treating, inhibiting or preventing secondary prostate tumor metastases.

9. A method for screening for a molecule that treats, inhibits or prevents a secondary prostate tumor metastasis comprising measuring the levels of complex formed from fit-4 and VEGF-C in the presence of a candidate molecule under conditions conducive to the formation of said complex; and comparing levels of said complex that are formed in the absence of the molecule, wherein a lower level of said complex in the presence of the molecule indicates that the candidate molecule has activity in treating, inhibiting or preventing secondary prostate tumor metastases.

10. A method for screening for a molecule that treats, inhibits or prevents a secondary prostate tumor metastasis comprising measuring the levels of complex formed from flt-4 and VEGF-D in the presence of a candidate molecule under conditions conducive to the formation of said complex; and comparing levels of said complex that are formed in the absence of the molecule, wherein a lower level of said complex in the presence of the molecule indicates that the candidate molecule has activity in treating, inhibiting or preventing secondary prostate tumor metastases.

11. A method of monitoring the efficacy of a method of treatment or inhibition of metastatic prostate cancer comprising measuring the level of expression or activity of flt-4 in prostate cells obtained from a subject wherein said sample is taken from said subject after the application of said method and compared to (a) said level in a sample taken from said subject prior to the application of said method or (b) a standard level associated with the pretreatment stage of metastatic prostate cancer, in which a decrease in the level of flt-4 expression or activity in said sample taken after application of said method relative to the level of flt-4 expression or activity in said sample taken before application of said method or to said standard level indicates that said method is effective.

12. A pharmaceutical composition comprising a protein, which protein comprises a fragment of flt-4, which fragment consists of at least the amino acid sequence set forth in SEQ ID NO:2, which protein acts as a competitive inhibitor of flt-4 binding to its ligand VEGF-C; and a pharmaceutically acceptable carrier.

13. The composition according to claim 12 in which the protein is soluble.

14. A pharmaceutical composition comprising a nucleic acid, which nucleic acid comprises a nucleotide sequence which encodes for a fragment of flt-4, which fragment acts as a competitive inhibitor of flt-4 binding to its ligand VEGF-C; and a pharmaceutically acceptable carrier.

15. A pharmaceutical composition comprising an antisense oligonucleotide, which oligonucleotide comprises a nucleotide sequence consisting of at least 6 contiguous nucleotides complementary to the nucleotide sequence set forth in SEQ ID NO: 1; and a pharmaceutically acceptable carrier.

16. The composition according to claim 15 in which the antisense oligonucleotide comprises the nucleotide sequence 5'-GGCGCCCCGCTGCAT-3' (SEQ ID NO:3).

17. A pharmaceutical composition comprising an antibody or a portion thereof that binds to flt-4; and a pharmaceutically acceptable carrier.
